# EUROPEAN PATENT SPECIFICATION

(11) **EP 3 311 165 B1**
(45) Date of publication and mention of the grant of the patent: **09.12.2020**
(21) Application number: 16734800.2
(22) Date of filing: 17.06.2016
(51) Int. Cl.: G01N 33/574, A61P 35/00, A61K 38/00

(54) **SEPTIN PROTEINS AS NOVEL BIOMARKERS FOR DETECTION AND TREATMENT OF MÜLLERIAN CANCERS**
SEPTINPROTEINE ALS NEUARTIGE BIOMARKER ZUR DETEKTION UND BEHANDLUNG VON MÜLLERSCHEN TUMOREN
PROTÉINES SEPTINES EN TANT QUE NOUVEAUX MARQUEURS BIOLOGIQUES POUR LA DÉTECTION ET LE TRAITEMENT DE CANCERS DU SYSTÈME MÜLLERIEN

(30) Priority: 19.06.2015 US 201562182011 P
(43) Date of publication of application: 25.04.2018
(73) Proprietor: University Of Rochester, Rochester, NY 14642 (US)
(72) Inventor: MOORE, Richard, G., Victor, NY 14564 (US); SINGH, Rakesh, K., Barrington, RI 02806 (US)
(74) Representative: Lahrtz, Fritz
(86) International application number: PCT/US2016/038127
(87) International publication number: WO 2016/205681

(56) References cited:
- WO-A1-2011/109440
- WO-A2-2005/039487
- WO-A2-2012/112685
- MICHAEL SCOTT ET AL: "Altered patterns of transcription of the septin gene, SEPT9, in ovarian tumorigenesis", INTERNATIONAL JOURNAL OF CANCER, vol. 118, no. 5, 13 September 2005 (2005-09-13), pages 1325-1329, XP055303877, US ISSN: 0020-7136, DOI: 10.1002/ijc.21486
- SCOTT MICHAEL ET AL: "Multimodality expression profiling shows SEPT9 to be overexpressed in a wide range of human tumours", ONCOGENE, NATURE PUBLISHING GROUP, GB, vol. 24, no. 29, 7 July 2005 (2005-07-07), pages 4688-4700, XP002462084, ISSN: 0950-9232, DOI: 10.1038/SJ.ONC.1208574
- ZONGMING FU ET AL: "Proteomic Evidence for Roles for Nucleolin and Poly[ADP-ribosyl] Transferase in Drug Resistance", JOURNAL OF PROTEOME RESEARCH., vol. 4, no. 5, 1 October 2005 (2005-10-01) , pages 1583-1591, XP055303971, US ISSN: 1535-3893, DOI: 10.1021/pr0501158

## Description

### CROSS-REFERENCE TO RELATED APPLICATIONS

This application claims priority to U.S. Provisional Patent Application Publication No. 62/182,011, filed June 19, 2015.

### FIELD OF INVENTION

This invention relates, *inter alia,* to septin family proteins as novel biomarkers for the detection of Müllerian cancers as well as therapeutic targeting of septin-2 and other septin family genes and proteins for the treatment of cancer.

### BACKGROUND

Müllerian cancers, such as ovarian cancer, are most often diagnosed in an advance stage of the disease due to the lack of specific and sensitive biomarkers or due to the absence of clinical symptoms that would indicate the presence of small tumors. For example, approximately 70% of women afflicted with ovarian cancer are not diagnosed until distant metastases are present. The survival of patients with ovarian cancer directly correlates with the stage at which the disease is diagnosed. More than 90% of women diagnosed with ovarian cancer in stage I survive for at least 5 years following diagnosis. The 5-year survival rate drops to less than 30% when the disease is at stage III and IV. Similarly, uterine malignancies, including type I and II endometrial cancers, are the most frequently diagnosed gynecologic malignancies in the US. Type II endometrial cancers (high grade endometrioid, serous, clear cell and undifferentiated) often present with advanced stage disease where the disease is fundamentally incurable with 5 year survival rates below 20%. Even for patients diagnosed with early stage Type II endometrial malignancy, many will require adjuvant treatment in the form of chemotherapy, radiation therapy or combination chemo-radiation.

The development of biomarkers for the management of women with Müllerian cancers is critical for the diagnosis, prognosis, and management of their disease. Various studies directed at gene expression analysis have identified a series of potential biomarkers for evaluation in diagnostic applications have been described in literature (Ono et al. (2000) Cancer Res. 60:5007-11; Welsh et al. (2001) Proc. Natl. Acad. Sci. USA 98:1176-1181; Donninger et al. (2004) Oncogene 23:8065-8077; and Lee et al. (2004) Int. J. Oncol. 24(4):847-851). However, despite advancements in biomarker technology, there continues to be a need for sensitive biomarkers for the early detection of Müllerian cancers which could result in a stage migration to lower stages of disease where survival and cure rates are highest.

Throughout this specification, various patents, patent applications and other types of publications (*e.g*., journal articles, electronic database entries, *etc.*) are referenced.

### SUMMARY

The invention is defined in the claims and essentially relates to septin-2 as biomarker or therapeutic target for Müllerian cancer in the methods, uses and products disclosed herein. Subject-matter preceded or accompanied by terminologies such as "provided herein", "embodiment(s)" or relating to further septin biomarkers/targets can only form part of the invention in as far as they fall within the scope of the claims. If subject-matter falls outside the scope of the claims, it is to be considered as merely part of the general disclosure.

Provided herein, *inter alia,* are septin family proteins for use as novel biomarkers for the detection of Müllerian cancers such as ovarian, fallopian tube, primary peritoneal, endometrial, and uterine cancers (as well as well as all histologic sub types associated with the same) and methods for therapeutic targeting of septin-2 and other septin family genes and proteins via small molecule, antisense or antibody targeted therapies for treatment of the same. Accordingly, in some aspects, provided herein are methods for diagnostically evaluating a subject for a Müllerian cancer, the method comprising: measuring the expression of a septin family gene or protein or fragment thereof in a sample from the subject, wherein the subject is diagnosed with a Müllerian cancer if the expression of the septin family gene or protein or fragment thereof is higher in the sample than in one or more control samples acquired from one or more subjects without a Müllerian cancer. In some embodiments, the method further comprises measuring the expression of one or more biomarkers selected from CA125, cyclin dependent kinase inhibitor (p21), HE4, transthyretin, B2-Microglobulin, apolipoprotein A-1, prealbumin, transferrin, LPA, YKL-40, follicle stimulating hormone (FSH), anti-Müllerian Hormone (AMH) and/or inhibin, wherein the subject is diagnosed with a Müllerian cancer if a) the expression of the septin family gene or protein or fragment thereof and b) the expression of the one or more biomarkers is higher in the sample than in the one or more control samples acquired from one or more subjects without a Müllerian cancer. In some embodiments, the subject is pre- or post-menopausal. In some embodiments of any of the embodiments disclosed herein, the subject has been diagnosed as having a Müllerian cancer and the method is used to determine if the Müllerian cancer has recurred or advanced. In some embodiments of any of the embodiments disclosed herein, the subject has not been previously diagnosed as having a Müllerian cancer and the method is used to evaluate whether a Müllerian cancer is present. In some embodiments of any of the embodiments disclosed herein, the expression of the septin family gene or protein or fragment thereof in the sample is at least 1-500 times (such as 1-500 fold) higher than in the one or more control samples. In some embodiments of any of the embodiments disclosed herein, the Müllerian cancer is selected from the group consisting of ovarian cancer, fallopian tube cancer, primary peritoneal cancer, uterine cancer and endometrial cancer. In some embodiments, the septin family gene or protein or fragment thereof is septin-2. In some embodiments, the septin-2 gene or protein or fragment thereof in the sample is at least 3-6 folds higher compared to one or more control samples. In some embodiments, the expression of the septin-2 gene or protein or fragment thereof in the sample is at least 3-6 folds higher compared to one or more control samples and the expression of the HE4 gene or protein or fragment thereof in the sample is at least 4-8 folds higher compared to one or more control samples. In some embodiments, the expression of the septin-2 gene or protein or fragment thereof in the sample is at least 3-6 folds higher compared to one or more control samples and the expression of one or more of CA125, p21, transthyretin, LPA, YKL-40 and/or inhibin gene or protein or fragment thereof in the sample is at least 4-8 folds higher compared to one or more control samples. In another embodiment, provided herein are methods for diagnostically evaluating a subject for a Müllerian cancer, the method comprising: measuring the expression of a septin-2 gene or protein or fragment thereof in a sample from the subject, wherein the subject is diagnosed with a Müllerian cancer if the expression of the septin-2 gene or protein or fragment thereof is higher (such as at least about 3-6 folds higher) in the sample than in one or more control samples acquired from one or more subjects without a Müllerian cancer.

In another aspect, provided herein are methods for stratifying a subject with a pelvic mass for the risk of having or developing a Müllerian cancer, the method comprising measuring the expression of a septin family gene or protein or fragment thereof in a sample from the subject, wherein the subject is at increased risk for having or developing a Müllerian cancer if the expression of the septin family gene or protein or fragment thereof is higher in the sample than in one or more control samples acquired from one or more subjects without a Müllerian cancer. In some embodiments, the method further comprises measuring the expression of one or more biomarkers selected from CA125, HE4, transthyretin, B2-Microglobulin, apolipoprotein A-1, prealbumin, transferrin, LPA, YKL-40, follicle stimulating hormone (FSH), anti-Müllerian Hormone (AMH), cyclin dependent kinase inhibitor (p21), and/or inhibin, wherein the subject is at increased risk for having or developing a Müllerian cancer if a) the expression of the septin family gene or protein or a fragment thereof and b) the expression of the one or more biomarkers is higher in the sample than in the one or more control samples acquired from one or more subjects without a Müllerian cancer. In some embodiments of any of the embodiments disclosed herein, a) increased expression of the septin family gene or protein or fragment thereof or b) increased expression of the septin family gene or protein or fragment thereof expression and increased expression of the one or more biomarkers signifies that the subject is at increased risk for one or more of malignant cancer or early stage malignant cancer. In some embodiments of any of the embodiments disclosed herein, a) equivalent expression of the septin family gene or protein or fragment thereof or b) equivalent expression of the septin family gene or protein or fragment thereof and equivalent expression of the one or more biomarkers signifies that the pelvic mass is benign or a low malignant potential tumor (LMP). In some embodiments of any of the embodiments disclosed herein, the Müllerian cancer is selected from the group consisting of ovarian cancer, fallopian tube cancer, primary peritoneal cancer, uterine cancer and endometrial cancer.

In other aspects, provided herein are methods for classifying a pelvic mass in a subject at risk of having or developing a Müllerian cancer, the method comprising measuring the expression of a septin family gene or protein or fragment thereof in a sample from the subject, wherein the pelvic mass is classified as being malignant if the expression of the septin family gene or protein or fragment thereof is higher in the sample than in one or more control samples acquired from subjects without a Müllerian cancer. In some embodiments, the method further comprises: (i) measuring the quantity of CA125 and/or HE4 in the sample; (ii) comparing the quantity of the septin family gene or protein or fragment thereof and the quantity of CA125 and/or HE4 as measured in (i) with a reference value of the quantity of the septin family gene or protein or fragment thereof and the quantity of CA125 and/or HE4, wherein the reference value represents a known classification of a pelvic mass, Müllerian tumor, or Müllerian cancer; (iii) finding a deviation or no deviation of the septin family gene or protein or fragment thereof and the quantity of CA125 and/or HE4 as measured in (i) from the reference value in (ii); and (iv) classifying the pelvic mass in the subject as being benign or malignant based on the finding of deviation or no deviation. In some embodiments, the method further comprises (i) measuring the quantity of (a) a septin family protein or fragment thereof, and (b) HE4 and/or CA125 in a sample from the subject; (ii) entering the quantities measured in (i) into an equation wherein each quantity is given a weight; and (iii) analyzing whether the numerical value obtained in (ii) falls within a range of benign to malignant Müllerian cancer, wherein the ranges of benign, early malignant, borderline, or malignant Müllerian cancer have been established by using the same equation on samples from subjects for which respectively benign or malignant Müllerian cancer has been previously diagnosed or classified. In some embodiments, the malignant Müllerian cancer is early malignant, or borderline. In some embodiments of any of the embodiments disclosed herein the method further comprises: measuring the quantity of one or more other biomarkers in the sample from the subject, wherein the other biomarker is one or more biomarkers selected from the group consisting of mesothelin (SMRP), transthyretin, B2-Microglobulin, apolipoprotein A-1, prealbumin, transferrin, LPA, YKL-40, follicle stimulating hormone (FSH), anti-Müllerian Hormone (AMH) and/or inhibin, and fragments or precursors of any one thereof. In some embodiments of any of the embodiments disclosed herein the method further comprises: morphologically analyzing the pelvic mass by a method selected from the group consisting of one or more of ultrasound (US), magnetic resonance imaging (MRI), computed tomography (CT), and positron emission tomography-computed tomography (PET-CT). In some embodiments of any of the embodiments disclosed herein the method further comprises: assessing risk factors in the subject selected from the group consisting of genetic predisposition due to mutations in the BRCA gene family, familial predisposition, age, diet, obesity, reproductive history, menopausal status, gynecological surgery, hormonal replacement therapy, smoking and alcohol use. In some embodiments gynecological surgery comprises tubal ligation or hysterectomy. In some embodiments of any of the embodiments disclosed herein, the method is used at regular time points to follow the expression of a) the septin family gene or protein or fragment thereof and/or b) the septin family gene or protein or fragment thereof and the biomarker(s) in combination with the risk factors during the life of the subject. In some embodiments of any of the embodiments disclosed herein, the septin family gene or protein or fragment thereof is septin-2. In some embodiments of any of the embodiments disclosed herein, the Müllerian cancer is selected from the group consisting of ovarian cancer, fallopian tube cancer, primary peritoneal cancer, uterine cancer and endometrial cancer.

In further aspects, provided herein are methods for detecting a change in the prognosis for a subject diagnosed with a Müllerian cancer comprising measuring the expression of a septin-2 gene, protein, or fragment thereof in the subject during or after treatment for the Müllerian cancer, wherein a change in the expression level of septin-2 in comparison with a reference value for expression of a septin-2 gene, protein, or fragment thereof in one or more subjects with benign tumors indicates a change in the prognosis for the subject. In some embodiments, the subject is pre- or post-menopausal. In some embodiments of any of the embodiments disclosed herein, the sample is blood, serum, plasma, or urine. In some embodiments of any of the embodiments disclosed herein, the Müllerian cancer or Müllerian tumor is selected from the group consisting of one or more of an epithelial carcinomas, malignant sex cord stromal tumor, malignant germ cell tumors, metastatic carcinoma infiltrated in the pelvis or in the ovaries, cystadenoma, fibroma, thecoma, cystadenofibroma, mature teratoma, endometriosis, follicular cyst, abscess, struma ovarii, Leydig cell tumor, parasalpingeal cyst, hydrosalpinx, corpus luteum cyst, hemorrhagic cyst, tissue with calcifications NOS, necrotic tumor NOS or combinations thereof. In some embodiments of any of the embodiments disclosed herein, septin family protein or a fragment thereof expression is measured. In some embodiments, the septin family protein or a fragment thereof expression is measured by immunohistochemistry, ELISA, RIA, Western or immunoblot, or another antibody-based method. In some embodiments, the septin family protein or a fragment thereof expression is measured by mass spectrometry or chromatography. In some embodiments of any of the embodiments disclosed herein, septin family gene expression is measured. In some embodiments, septin family gene expression is measured by qRT-PCR, RT- PCR or another PCR-based method, Northern Blot or SAGE. In some embodiments, DNA methylated forms of septin family genes, isoforms of septin family genes, circulating septin family DNA, or microRNA or fragments thereof are measured. In some embodiments of any of the embodiments disclosed herein, the Müllerian cancer is selected from the group consisting of ovarian cancer, fallopian tube cancer, primary peritoneal cancer, uterine cancer and endometrial cancer.

In yet other aspects, provided herein are methods for treating a proliferative disease in a subject comprising inhibiting the expression or activity of a septin family member gene, protein, or fragment thereof.

In some embodiments the inhibition results in an antitumor, anticancer, antiproliferative, anti-angiogenic, or anti-lipogenic effect.

In some embodiments of any of the embodiments disclosed herein, septin family member gene expression is inhibited by administration of an effective amount of one or more agents selected from the group consisting of a small molecule chemical compound, an antisense oligonucleotide, a siRNA, a phosphorothio oligonucleotide (PTOs).

In some embodiments of any of the embodiments disclosed herein, septin family member protein or fragment thereof expression is inhibited by administration of an effective amount of one or more agents selected from the group consisting of an antibody or fragment thereof, a small molecule chemical compound, and a non-antibody peptide.

In some embodiments of any of the embodiments disclosed herein, the method further comprises administering to the subject one or more of cytotoxic, cytostatic, antiangiogenic, anti-tyrosine kinase inhibitor, cytoreduction, irradiation, plant, or food based therapies.

In another aspect, provided herein are kits comprising (i) means for measuring (a) the quantity of septin-2 or other septin family proteins or fragments thereof and (b) the quantity of CA125, HE4 and/or one or more other biomarkers in a sample from a subject; and (ii) a reference value of (a) the quantity of septin-2 or other septin family proteins or a fragment thereof and (b) the quantity of CA125, HE4 and/or one or more other, wherein the reference value represents a known classification of a Müllerian tumor. In some embodiments of any of the embodiments disclosed herein, the Müllerian tumor is selected from the group consisting of ovarian cancer, fallopian tube cancer, primary peritoneal cancer, uterine cancer and endometrial cancer.

Each of the aspects and embodiments described herein are capable of being used together, unless excluded either explicitly or clearly from the context of the embodiment or aspect.

### BRIEF DESCRIPTION OF THE DRAWINGS

FIG. 1 illustrates the relative expression of septin-2 in normal, benign and various phenotypes of ovarian cancer. Serous high stands for serous EOC tissues with high HE4 expression. Serous low stands for serous EOC tissues presenting low HE4 expression. Clear cell high and clear cell low represents clear cell EOC tissues with high and low HE4 expressions respectively. Similarly, endometrioid high and low stands for the endometrioid EOC tissues with high and low HE4 expressions. The relative expression of septin-2 was measured in Integrated Optical Density (IOD) units.

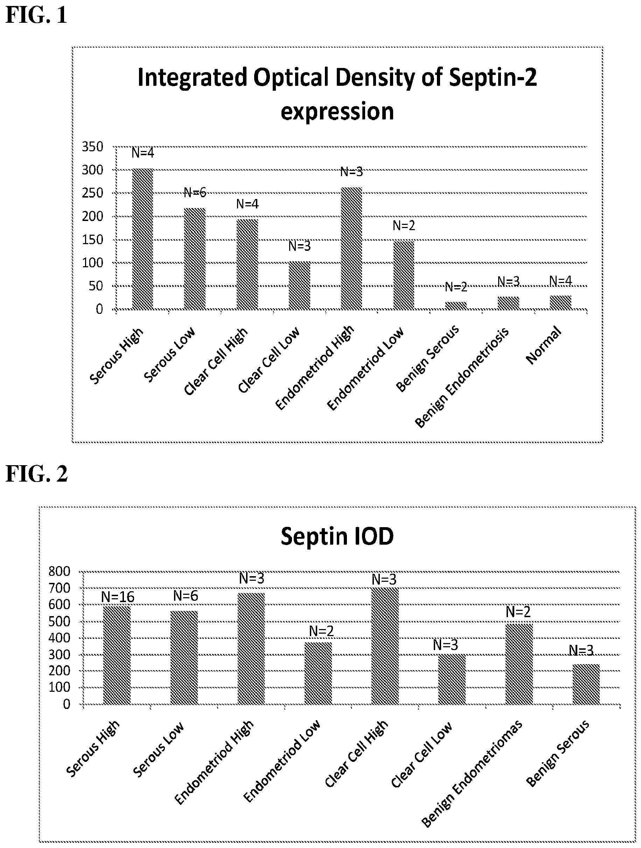

FIG. 2 illustrates the relative expression of septin-2 in normal, benign and various phenotypes of additional number of ovarian cancer patient derived tissues. Serous high stands for serous EOC tissues with high HE4 expression. Serous low stands for serous EOC tissues presenting low HE4 expression. Clear cell high and clear cell low represents clear cell EOC tissues with high and low HE4 expressions respectively. Similarly, endometrioid high and low stands for the endometrioid EOC tissues with high and low HE4 expressions. The relative expression of septin-2 was measured in Integrated Optical Density (IOD) units.
FIG. 3 illustrates the relative expression of septin-2 in benign and serous ovarian cancer patient derived tissues. Serous high stands for serous EOC tissues with high HE4 expression. Serous low stands for serous EOC tissues presenting low HE4 expression. The relative expression of septin-2 was measured in Integrated Optical Density (IOD) units.
FIG. 4 illustrates the relative expression of septin-2 in benign and ovarian cancer patient derived tissues. The relative expression of septin-2 was measured in Integrated Optical Density (IOD) units.
FIG. 5 illustrates the co-localization of septin-2 and HE4 and describes the integrated optical density measurements of normal, benign and ovarian cancer tissue phenotypes.
FIG. 6 illustrates cytotoxic effects of library of septin-2 targeting phosphorothio-oligos (PTOs) in platinum resistant SKOV-3 ovarian cancer cells.
FIG. 7 illustrates cytotoxic effects of Septin-2 targeting phosphorothio-oligos (PTOs) in platinum resistant OVCAR-8 ovarian cancer cells.
FIG. 8 depicts a western blot (top) showing septin-2 protein levels in ovarian cancer cells engineered to stably express shRNAs against septin-2 as well as a bar graph showing the proliferation of these cells following 72 and 92 hours in culture (bottom).
FIG. 9 depicts a confocal fluorescent micrograph showing that overexpression of HE4 leads to septin-2 activation (left). Stable HE4 expressing SKOV-3 cells clones (clone-1 and clone-2) along with wild-type and null vector were fixed and stained with septin-2 antibody and corresponding Alexa Fluor antibody. The confocal images were acquired. A western blot (left) shows that dividing cells express elevated septin-2 among the cells that stably overexpress HE4.
FIG. 10 depicts a confocal fluorescent micrograph showing that overexpression of HE4 leads to septin-2 activation. Stable HE4 expressing OVCAR-8 cell clones (clone-5) along with wild-type and null vector were fixed and stained with septin-2 antibody and corresponding Alexa Fluor antibody.
FIG. 11 depicts SDS-PAGE gels showing the results of immunoprecipitation experiments. SKOV3 WT and OVCAR-8 cells lysates were immunoprecipitated with pull-down antibody (septin-2 (left) or HE4 (right)) and isotype-matched IgG control, with antibody concentration identical at 40 ng/µl. Further processing and PAGE analysis of samples was conducted to resolve the bands.
FIG. 12 depicts SDS-PAGE gels showing the results of immunoprecipitation experiments showing that immunoprecipitation of septin-2 captures p21. SKOV3 WT and OVCAR-8 cells lysates were immunoprecipitated with pull-down antibody (septin-2) and isotype-matched IgG control, with antibody concentration identical at 40 ng/µl. Further processing and PAGE analysis of samples was conducted to resolve the bands and to detect p21.

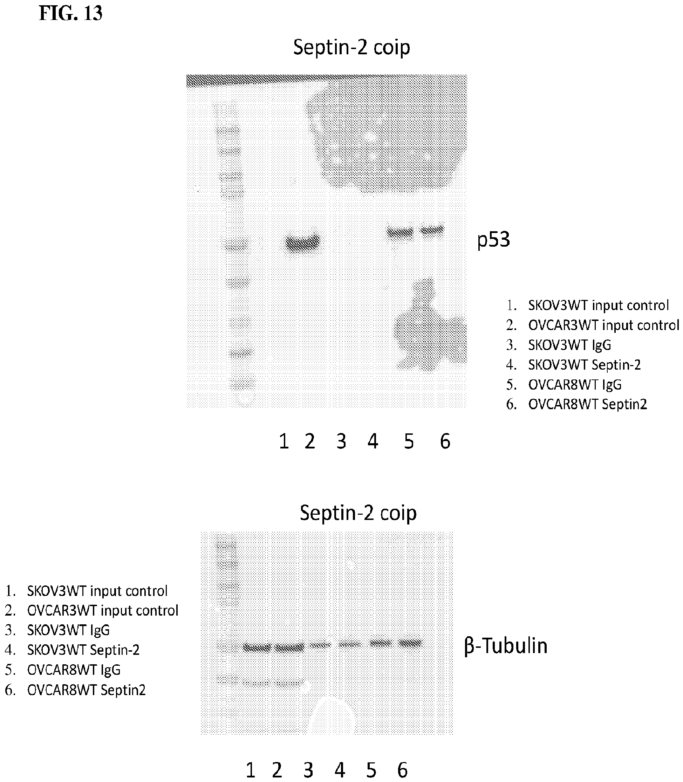

FIG. 13 depicts SDS-PAGE gels showing the results of immunoprecipitation experiments showing that immunoprecipitation of septin-2 did not capture p53 or β-tubulin.
FIG. 14 depicts the results of an ovarian tumor tissue array fluorescently stained with a septin-2 specific antibody. Tissue from normal ovary, serous adenocarcinoma, mucinous adenocarcinoma, clear cell carcinoma, and dysgerminoma were examined (bottom) and florescent intensity quantified (top).
FIG. 15 illustrates the relative expression of septin-2 in clear cell ovarian carcinoma tissue versus expression in tissue derived from benign endometriotic, benign serous, and normal ovarian tissue.
FIG. 16 depicts a bar graph illustrating relative septin-2 intensity in tissue sections derived from clear cell ovarian carcinoma, benign endometriotic, benign serous, and normal ovarian tissue.

### DETAILED DESCRIPTION

There are currently no accepted screening regimens for the early detection of Müllerian cancers. Paradoxically, the low prevalence rates of Müllerian cancers (such as ovarian cancer) in the general population create significant challenges for the development of a screening test that would promote early detection of the disease. Screening methods for diseases with low prevalence rates (such as ovarian cancer) often result in a high ratio of false positives to true positives which limit the clinical utility of such screening programs. Given the significant risks associated with surgical exploration for possible Müllerian cancers, a clinically useful screening test should refer to surgery no more than 10 women for every woman who actually has a Müllerian cancer (i.e., a positive predictive value (PPV) of at least 10%). Skates et al. (2004) J. Clin. Oncol. 22:4059-4066. Currently available methods, such as detection of CA125 for the detection of ovarian cancer, exhibit unacceptably high false-positive rates.

The invention described herein provides, *inter alia,* novel biomarkers for the diagnosis, detection, and management of Müllerian cancers in women based on the selective expression of genes and/or proteins of members of the septin family (such as, but not limited to, septin-2). The inventors have discovered that septin family members are selectively overexpressed in serous, clear cell, and endometrioid histologic subtypes of ovarian cancer compared to normal ovarian tissue and benign neoplastic ovarian tissues. Further, septin family member expression is associated with HE4, regulates various oncogenic functions, and participates in angiogenesis and cell division that individually or in combination play role in ovarian oncogenesis. Additionally, disclosed herein are compositions and methods for treating Müllerian cancers (such as ovarian cancers) employing septin family member nucleic acid targeting antisense or phosphorothio- oligonucleotides (PTOs) which inhibit the cellular proliferation and growth of cancer cells and induce apoptosis or cell death.

### 1. Definitions

As used herein, the phrase "Müllerian cancer" indicates any cancer arising from any part of the female genital tract (such as, but not limited to, the uterus, fallopian tubes, ovaries and/or other female genital tract malignancies). In some embodiments, the term Müllerian cancer can refer to ovarian, fallopian tube, primary peritoneal, endometrial and uterine cancers, including all histologic sub types associated with the same, such as, but not limited to serous, endometrioid, clear cell, mucinous, undifferentiated, poorly differentiated, carcinosarcoma (MMMT), sarcoma germ cell tumors, and sex cord stromal tumors.

By "Müllerian tumor" or "ovarian tumor" it is meant any of epithelial carcinomas, malignant sex cord tumors, malignant germ cell tumors, metastatic carcinoma infiltrated in the pelvis or in the ovaries, cystadenoma, fibroma, thecoma, cystadenofibroma, mature teratoma, endometriosis, follicular cyst, abscess, struma ovarii, Leydig cell tumor, parasalpingeal cyst, hydrosalpinx, corpus luteum cyst, hemorrhagic cyst, tissue with calcifications NOS, necrotic tumor NOS or combinations thereof.

"Septin family members," as used herein, refers to a family of related GTP-binding proteins found primarily in eukaryotic cells of fungi and animals, but also in some green algae. Septins are P-Loop-NTPase proteins that range in weight from 30-65 kDa. Septins are highly conserved between different eukaryotic species. They are composed of a variable-length proline rich N-terminus with a basic phosphoinositide binding motif important for membrane association, a GTP-binding domain, a highly conserved Septin Unique Element domain, and a C-terminal extension including a coiled coil domain of varying length. In some embodiments, septin family members refer to any of septin-1, septin-2, septin-3, septin-5, septin-6, septin-7, septin-8, septin-9, septin-10, septin-11, septin-12, septin-13, or septin-14. In one embodiment, the septin family member is septin-2. In another embodiment, the septin-2 protein is represented by the amino acid sequence of SEQ ID NO:1. In another embodiment, the septin-2 protein is encoded by the nucleic acid sequence of SEQ ID NO:2.

As used herein, "risk stratification" refers to an estimation of the probability (*i.e.,* risk) that a subject has a clinical condition at the time a sample is taken from the subject.

As used herein, "classification of" and "classifying" a subject's cancer, tumor, or mass refer to determining one or more clinically-relevant features of the cancer or determining the prognosis of the subject. Thus "classifying a cancer" includes, but is not limited to: (i) evaluating metastatic potential, potential to metastasize to specific organs, risk of recurrence, or course of the tumor or mass; (ii) evaluating tumor stage; (iii) patient prognosis in the absence of therapy treatment of the cancer; (iv) prognosis of patient response to treatment (chemotherapy, radiation therapy, and/or surgery to excise the tumor or mass); (v) diagnosis of actual patient response to current and/or past treatment; (vi) determining a preferred course of treatment for the patient; (vii) prognosis for patient relapse after treatment; (viii) prognosis for patient survival.

As used herein, the term "protein" includes polypeptides, peptides, fragments of polypeptides, and fusion polypeptides.

As used herein, a "nucleic acid" refers to two or more deoxyribonucleotides and/or ribonucleotides covalently joined together in either single or double-stranded form.

A "subject" can be a vertebrate, a mammal, or a human. Mammals include, but are not limited to, farm animals, sport animals, companion animals such as pets, primates, mice and rats. In one aspect, a subject is a human.

The terms "treating" and "treatment" as used herein refer to the administration of an agent or formulation to a clinically symptomatic subject afflicted with an adverse condition, disorder, or disease, so as to effect a reduction in severity and/or frequency of symptoms, eliminate the symptoms and/or their underlying cause, and/or facilitate improvement or remediation of damage.

An "effective amount" or "therapeutically effective amount" refers to an amount of therapeutic compound, such as an oligonucleotide, small molecule, antibody, or any other anticancer therapy, administered to a subject, either as a single dose or as part of a series of doses, which is effective to produce a desired therapeutic effect.

The phrase "inhibiting the activity of a septin family member gene, protein, or fragment thereof," as used herein, means inhibiting one or more or all of the biological and/or biochemical functions of one or more septin family members (such as septin-2) without necessarily affecting (1) expression of the genes encoding one or more septin family member(s) and/or (2) expression of one or more septin family member proteins or fragments thereof.

The phrase "inhibiting the expression of a septin family member," as used herein, means inhibiting the expression one or more septin family member(s) (such as septin-2) at the level of DNA transcription into RNA or RNA translation into protein, thereby resulting in decreased or no septin family member RNA and/or protein in a cell. In some embodiments, inhibiting the expression of one or more septin family member(s) encompasses manipulating a cell to cause proteolytic degradation of one or more septin family member protein(s). In some embodiments, inhibiting the expression of one or more septin family member(s) encompasses manipulating a cell to cause degradation of one or more septin family member RNA(s).

As used herein, "fold higher" or "fold lower" or the synonymous phrases "times higher" or "times lower" change values refer to a numerical representation of the expression level of a gene, genes, gene fragments, or proteins encoded by one or more genes between experimental paradigms, such as a test or treated cell or tissue sample, compared to any standard or control (such as, without limitation, normal tissue or tissue with benign cellular growth). For instance, a fold higher change value may be presented as immunohistochemical or detectable probe intensities for a gene or genes from a test cell (such as an ovarian cell) or tissue (such as ovarian tissue) sample compared to a control, such as a normal cell or tissue sample or a vehicle-exposed cell or tissue sample.

Unless defined otherwise herein, all technical and scientific terms used herein have the same meaning as commonly understood by one of ordinary skill in the art to which this invention pertains.

As used herein, the singular terms "a," "an," and "the" include the plural reference unless the context clearly indicates otherwise.

The transitional term "comprising," which is synonymous with "including," "containing," or "characterized by," is inclusive or open-ended and does not exclude additional, unrecited elements or method steps. By contrast, the transitional phrase "consisting of excludes any element, step, or ingredient not specified in the claim. The transitional phrase "consisting essentially of limits the scope of a claim to the specified materials or steps "and those that do not materially affect the basic and novel characteristic(s)" of the claimed invention.

### II. Methods of the Disclosure

### A. Diagnosis of cancer

Effective methods for diagnosing a Müllerian cancer and for identifying subjects thought to have or with a likelihood of having a Müllerian cancer are provided herein. Methods for diagnosing cancer in a subject can encompass detecting the expression of a septin family member protein or nucleic acid (such as, but not limited to, septin-2) in a sample provided by the subject relative to one or more control samples derived from subjects who have not been diagnosed with cancer. In some embodiments, the septin family member (such as septin-2) is expressed at least 1-500 times (such as 1-500 fold) higher (such as any of about 1-450, 1-350, 1-250, 1-150, 1-100, 1-90, 1-80, 1-70, 1-60, 1-50, 1-40, 1-30, 1-20, 1-10, 1-9, 1-8, 1-7, 1-6, 1-5, 1-4, 1-3, 1-2, 1.3-6, 1.3-8, 2-3, 2-4, 2-5, 2-6, 2-7, 2-8, 2-9, 2-10, 2-12, 2-15, 3-4, 3-5, 3-6. 3-7, 3-8, 3-9, 3-10, 3-12, 3-15, 3-20, 4-5, 4-6, 4-7, 4-8, 4-9, 4-10, 4-12, 4-15, 4-20, 5-6, 5-7, 5-8, 5-9, 5-10, 5-12, 5-15, 5-20, 6-7, 6-8, 6-9, 6-10, 6-12, 6-15, or 6-20 fold higher) in the sample from the subject with a likelihood of having a Müllerian cancer compared to samples derived from subjects who do not have a Müllerian cancer (such as a sample characterized by normal or benign tissue growth). In other embodiments, the septin family member (such as septin-2) is expressed at least any of about 1, 1.1, 1.2, 1.3, 1.4, 1.5, 1.6, 1.7, 1.8, 1.9, 2, 2.1, 2.2, 2.3, 2.4, 2.5, 2.6, 2.7, 2.8, 2.9, 3, 3.1, 3.2, 3.3, 3.4, 3.5, 3.6, 3.7, 3.8, 3.9, 4, 4.1, 4.2, 4.3, 4.4, 4.5, 4.6, 4.7, 4.8, 4.9, 5, 5.1, 5.2, 5.3, 5.4, 5.5, 5.6, 5.7, 5.8, 5.9, 6, 6.1, 6.2, 6.3, 6.4, 6.5, 6.6, 6.7, 6.8, 6.9, 7, 8, 9, 10, 11, 12, 13, 14, 15, 16, 17, 18, 19, 20, 21, 22, 23, 24, 25, 30, 35, 40, 45, 50, 55, 60, 65, 70, 75, 80, 85, 90, 95, 100, 110, 120, 130, 140, 150, 160, 170, 180, 190, 200, 210, 220, 230, 240, 250, 275, 300, 325, 350, 375, 400, 425, 450, 475, or 500 times or fold higher in the sample from the subject with a likelihood of having a Müllerian cancer compared to samples derived from subjects who do not have a Müllerian cancer (such as a sample characterized by normal or benign tissue growth), inclusive of values falling in between these numbers. In one embodiment, the Müllerian cancer is ovarian cancer, such as clear cell ovarian carcinoma.

Septin family member protein or nucleic acid expression can be used as a biomarker for a Müllerian cancer (such as, but not limited to, ovarian cancer, uterine cancer, or endometrial cancer) by assessing the expression levels of a septin family member gene, protein or fragment thereof (such as, but not limited to, septin-2) in a biological sample from a subject or subpopulation of subjects suspected of having or developing a Müllerian cancer. As used herein, "septin family member gene or protein or fragment thereof expression" encompasses the existence of the full and intact septin family member DNA sequence (including, *e.g*., promoter elements, enhancer sequences, introns, and exons), the conversion of the septin family member DNA gene sequence into transcribed mRNA (including, *e.g*., the initial unspliced mRNA transcript or the mature processed mRNA), and/or the translated septin family member protein product (including, *e.g.* any posttranslational modifications such as, but not limited to, ubiquitination, sumoylation, acetylation, methylation, glycosylation, and/or hydroxylation).

Assessment of septin family member expression can be at the levels of protein, mRNA, or DNA. Assessment of mRNA expression levels of gene transcripts is routine and well known in the art. For example, one flexible and sensitive quantitative method for assessing mRNA expression levels in a biological sample is by quantitative RT-PCR (qRT-PCR) or by any other comparable quantitative PCR-based method. Additional methods for assessing septin family member mRNA expression include, but are not limited to, Northern blotting, microarrays, *in situ* hybridization, and serial analysis of gene expression (SAGE). In one embodiment, the septin family member is septin-2. In another embodiment, the septin-2 protein is encoded by the nucleic acid sequence of SEQ ID NO:2.

Similarly, assessments of septin family protein (or a fragment thereof) expression levels are routine in the art. For example, one method of measuring protein levels is via Western blotting or immunohistochemistry using commercially-available antibodies to septin family members (such as, but not limited to septin-2). However, without being bound to theory, there is a correlation between the expression level of a septin family member and the severity of or likelihood of developing cancer (such as, but not limited to, ovarian, fallopian tube, primary peritoneal, endometrial and uterine cancers). Consequently, the sensitivity of the protein assay is particularly important. Therefore, RIA, ELISA, flow cytometry, or any other more sensitive quantitative method of measuring septin family protein expression can be used instead of less quantitative methods. In one embodiment, the septin family member is septin-2. In another embodiment, the septin-2 protein is encoded by the amino acids of sequence of SEQ ID NO:1.

### Other Biomarkers

In addition to assessing the expression levels of septin family members (such as, but not limited to, septin-2), the expression levels of other biomarkers may also be used to diagnose a Müllerian cancer (such as, ovarian cancer). For example, rising serum levels of CA125 are associated with the presence of ovarian cancer. CA125 is a well characterized tumor marker normally expressed on the surface of epithelial cells and is detected in the serum of healthy women at low baseline levels. Elevated serum levels of CA125 can be detected in up to 80% of women with epithelial ovarian cancer. However only half of women with early stage disease (stage I) will have elevated serum levels of CA125 and approximately 20% of women with epithelial ovarian cancer will not have a marker for their disease. Currently CA125 has been cleared by the FDA only for monitoring women diagnosed with epithelial ovarian cancer. Clinically, serum CA125 measurements are used for monitoring disease status including response to treatment (progression and regression) and for detecting recurrent disease following treatment. However, the use of CA125 alone is ineffective for general population screening for ovarian cancer due to issues of limited sensitivity, specificity and low positive predictive value of <3%. (*see* Bast (2003) J Clin Oncol. 21(10 Suppl):200-205).

Additionally, HE4 (WFDC2) has been developed as a novel biomarker for the detection and monitoring ovarian cancer. This biomarker is overexpressed in epithelial ovarian cancer (EOC) and can be used for the clinical management of women diagnosed with this disease. HE4 has been cleared by the FDA for monitoring women diagnosed with epithelial ovarian cancer. The Risk of Ovarian Malignancy Algorithm (ROMA), which utilizes the biomarkers HE4 and CA125 along with menopausal status to determine the risk for ovarian cancer in a women diagnosed with a pelvic mass, has also been developed and can be used in conjunction with any of the diagnostic, prognostic, stratification, or classification methods disclosed herein (Moore et al., 2010, Am J Obstet Gynecol 2010;203:228.e1-6). Recently the ROMA using HE4 and CA125 received clearance from the United Stated Food and Drug Agency (USFDA) for risk assessment for ovarian cancer in women with an ovarian cyst or pelvic mass.

Further biomarkers useful for diagnosis, prognosis, stratification, and classification of a Müllerian cancer or pelvic masses as described by any of the methods disclosed herein include one or more of mesothelin (SMRP), transthyretin, B2-Microglobulin, apolipoprotein A-1, prealbumin, transferrin, LPA, YKL-40, cyclin dependent kinase inhibitor (p21), follicle stimulating hormone (FSH), anti-Müllerian Hormone (AMH) and/or inhibin.

Accordingly, in other embodiments of the methods of the disclosure, septin family member gene or protein or fragment thereof expression (such as, *e.g*., septin-2) and/or the quantity of CA125, HE4 and/or one or more other biomarkers may be measured by any suitable technique such as is known in the art. For example, the quantity of septin-2 protein or fragments thereof and/or the quantity of CA125, HE4 and/or one or more other biomarkers may be measured using, respectively, an antibody or other binding agent capable of specifically binding to a septin family member or fragments thereof, and a binding agent capable of specifically binding to CA125, HE4 and/or one or more other biomarkers. The binding agent may be, for example, an antibody, aptamer, photoaptamer, protein, peptide, peptidomimetic or a small molecule chemical compound. In one embodiment, the quantity of septin-2 protein or fragments thereof and/or the quantity of CA125, HE4 and/or one or more other biomarkers may be measured using an immunoassay technology, a mass spectrometry analysis method, a chromatography method, or a combination of these methods.

In some embodiments, a septin family member (such as septin-2) and/or one or more other biomarkers (such as, without limitation, HE4, CA125, SMRP, transthyretin, B2-microglobulin, apolipoprotein A-1, prealbumin, transferrin, LPA, YKL-40, FSH, AMH and/or inhibin) can be used for detecting or diagnosing a Müllerian cancer (such as ovarian cancer, for example, clear cell ovarian carcinoma) in a sample provided by a subject thought to have or who is at risk of developing a Müllerian cancer relative to one or more control samples derived from subjects who have not been diagnosed with a Müllerian cancer. In some embodiments, the septin family member (such as septin-2) and/or one or more other biomarkers are expressed at least 1-500 fold higher (such as any of about 1-450, 1-350, 1-250, 1-150, 1-100, 1-90, 1-80, 1-70, 1-60, 1-50, 1-40, 1-30, 1-20, 1-10, 1-9, 1-8, 1-7, 1-6, 1-5, 1-4, 1-3, 1-2, 2-3, 2-4, 2-5, 2-6, 2-7, 2-8, 2-9, 2-10. 2-12, 2-15, 3-4, 3-5, 3-6, 3-7, 3-8, 3-9, 3-10, 3-12, 3-15, 3-20, 4-5, 4-6, 4-7, 4-8, 4-9, 4-10, 4-12, 4-15, 4-20, 5-6, 5-7, 5-8, 5-9, 5-10, 5-12, 5-15, 5-20, 6-7, 6-8, 6-9, 6-10, 6-12, 6-15, or 6-20 fold higher) in the sample from the subject thought to have or to be at risk of developing a Müllerian cancer compared to samples derived from subjects who do not have a Müllerian cancer (such as a sample characterized by normal or benign tissue growth). In other embodiments, the septin family member (such as septin-2) and/or one or more other biomarkers are expressed at least any of about 1, 1.1, 1.2, 1.3, 1.4, 1.5, 1.6, 1.7, 1.8, 1.9, 2, 2.1, 2.2, 2.3, 2.4, 2.5, 2.6, 2.7, 2.8, 2.9, 3, 3.1, 3.2, 3.3, 3.4, 3.5, 3.6, 3.7, 3.8, 3.9, 4, 4.1, 4.2, 4.3, 4.4, 4.5, 4.6, 4.7, 4.8, 4.9, 5, 5.1, 5.2, 5.3, 5.4, 5.5, 5.6, 5.7, 5.8, 5.9, 6, 6.1, 6.2, 6.3, 6.4, 6.5, 6.6, 6.7, 6.8, 6.9, 7, 8, 9, 10, 11, 12, 13, 14, 15, 16, 17, 18, 19, 20, 21, 22, 23, 24, 25, 30, 35, 40, 45, 50, 55, 60, 65, 70, 75, 80, 85, 90, 95, 100, 110, 120, 130, 140, 150, 160, 170, 180, 190, 200, 210, 220, 230, 240, 250, 275, 300, 325, 350, 375, 400, 425, 450, 475, or 500 fold higher in the sample from the subject thought to have or to be at risk of developing a Müllerian cancer compared to samples derived from subjects who do not have a Müllerian cancer (such as a sample characterized by normal or benign tissue growth), inclusive of values falling in between these numbers.

In some embodiments, septin family protein expression (such as septin-2 protein expression) is assessed via a two-antibody or "sandwich" ELISA technique, as described herein. The methods of the disclosure encompass, for example, a "two-step" analysis, wherein a first assay step is performed to detect the expression of a first biomarker (*e.g*., septin-2 other septin family protein) or panel of biomarkers such as, but not limited to, one or more of CA125, HE4, transthyretin, B2-Microglobulin, apolipoprotein A-1, prealbumin, transferrin, LPA, YKL-40, follicle stimulating hormone (FSH), anti-Müllerian Hormone (AMH) and/or inhibin. If the first biomarker or panel of biomarkers is overexpressed, a second assay step is performed to detect the expression of a second biomarker or panel of biomarkers. Overexpression of the first and second biomarkers or panels of biomarkers in sum total can function as an indicator of increased likelihood that a subject has a Müllerian cancer. The methods of the disclosure can utilize the disclosed septin-2 antibodies to detect expression of septin-2 or other septin family proteins in a subject's sample.

In another embodiment a septin family member protein (such as, *e.g*., septin-2) or fragments thereof and/or the quantity of CA125, HE4 and/or one or more other biomarkers is measured using an immunoassay technology, or using a mass spectrometry analysis method or using a chromatography method, or using a combination of these methods.

The methods disclosed herein may be used to diagnose any form of Müllerian cancer disclosed herein (such as, but not limited to, ovarian, fallopian tube, primary peritoneal, endometrial and uterine cancers as well as various phenotypes associated with the same). The methods disclosed herein may also be used to diagnose endometrial or uterine cancers.

### B. Classification and stratification of Müllerian tumors

Also disclosed herein are methods for the classification of a Müllerian tumor or pelvic mass in a subject based on the expression levels of a septin family member gene, protein, or fragment thereof (such as, septin-2) and HE4 or other biomarkers individually or in combination compared to the levels of these biomarkers in one or more control subjects who do not have Müllerian tumors and/or pelvic masses. The classifying step in any of these methods can include assigning the Müllerian tumor or pelvic mass to a tumor subclass.

The tumor subclass may be selected from any of epithelial carcinoma, endometrioid epithelial carcinoma, mucinous epithelial carcinoma, clear cell epithelial carcinoma, Brenner epithelial carcinoma, carcinosarcoma, undifferentiated epithelial carcinoma, granulosa sex cord carcinoma, Sertoli-Leydig sex cord carcinoma, gynandroblastoma, dysgerminoma, yolk sac carcinoma, embryonal carcinoma, choriocarcinoma, immature teratoma, serous cystadenoma, endometrioid cystadenoma, mucinous cystadenoma, clear cell cystadenoma, Brenner cystadenoma, fibroma, thecoma, fibrothecoma, serous cystadenofibroma, mucinous cystadenofibroma, clear cell cystadenofibroma, Brenner cystadenofibroma, mature teratoma, endometriosis, endometrioma, follicular cyst, abscess (pelvic inflammatory disease), struma ovarii, Leydig cell tumour, parasalpingeal cyst, hydrosalpinx, corpus luteum cyst, hemorrhagic cyst, paratubal cyst, tissue with calcifications NOS, necrotic tumor NOS and combinations thereof.

Any of the methods described herein for classifying a Müllerian tumor in a subject can encompass: (i) measuring the quantity of one or more septin family (such as, septin-2) nucleic acids, proteins or fragments thereof and the quantity of CA125 and HE4 in a sample from the subject; (ii) using the measurements of (i) to establish a subject profile of the quantity one or more septin family proteins or fragments thereof and the quantity of CA125 and HE4; (iii) comparing the subject profile of (ii) to a reference profile of the quantity of one or more septin family proteins or fragments thereof and the quantity of CA125 and HE4, the reference profile representing a known classification of a Müllerian tumor; and (iv) finding a deviation or no deviation of the subject profile of (ii) from the classification in the reference profile. In some embodiments, the classification method may also include a step of (v) classifying the Müllerian tumor in the subject as being benign or malignant based on the finding of deviation or no deviation. In other embodiments, the Müllerian tumor in the subject is classified as being malignant based on a deviation of any of 5%, 10%, 15%, 20%, 25%, 30%, 35%, 40%, 45%, 50%, 55%, 60%, 65%, 70%, 75%, 80%, 85%, 90%, 95%, 100%, or more (inclusive of values falling in between these percentages) compared to the reference profile.

Also provided herein are methods for the stratification of a Müllerian tumor in a subject. These methods can include the steps of: (i) measuring the quantity of one or more septin family(such as, septin-2) nucleic acids, proteins, or fragments thereof, optionally compensated by the measured EGFR value, and measuring the quantity of HE4 and/or CA125 nucleic acids, proteins, or fragments thereof in a sample from the subject; (ii) entering the quantities measured in (i) into an equation; and (iii), analyzing whether the numerical value obtained in (ii) falls within the range of 1) benign or 2) malignant Müllerian tumor, preferably within the range of 1) benign or 2) early malignant, or borderline (LMP) tumors.

The ranges of numerical values produced by the equation that reflect benign or malignant (for example, early stage malignant, or borderline) Müllerian cancer, can be established by measuring the quantity of one or more septin family proteins (such as, septin-2) or fragments thereof, optionally compensated by the measured expression levels of EGFR, VEGFR, interleukins, TNF1a or other soluble factors or hormones, and measuring the quantity of HE4 and/or CA125 in a sample from a subject for which the Müllerian cyst, tumor or cancer has been previously diagnosed or classified.

In one embodiment of any of the methods disclosed herein, classifying of tumors includes distinguishing benign tumors from malignant cancers. As such, benign tumors can be distinguished from early stage Müllerian cancers as well as low malignant potential tumors (LMPs).

In addition, classifying or stratifying tumors can also include or be combined with clinical parameters for evaluating the malignancy or risk thereto of pelvic masses. In particular ultrasound, (US), magnetic resonance imaging (MRI), computed tomography (CT), positron emission tomography-computed tomography (PET-CT) can be used to assess the morphology of the cyst(s), and/or the presence of multi-locular cysts, evidence of solid areas, evidence of a complex pelvic mass, evidence of an adnexal mass, evidence of metastases, presence of ascites, and/or bilateral lesions. Additionally, the age or menopausal status of the subject can be taken into account. The Risk of Malignancy Index (RMI) or ROMA for example combines these clinical parameters with the serum level of CA125 in the subject resulting in a risk score. Combining said test with the assessment of the blood, serum or plasma expression level of one or more septin family genes, proteins, or fragments thereof (such as, septin-2) can also be performed.

### C. Monitoring subjects post-surgery or post-chemotherapy

In some aspects, also provided herein is a method for monitoring a subject with a Müllerian cancer post-surgery. In some embodiments, the method encompasses (i) measuring the quantity of a septin family member's (such as, septin-2) nucleic acid or protein (or fragments thereof) expression and the quantity of CA125 and/or HE4 nucleic acid or protein expression in a sample provided by the subject; (ii) using the measurements of (i) to establish a subject profile of the quantity of septin family protein expression or fragments thereof and the quantity of CA125 and/or HE4; (iii) comparing said subject profile of (ii) to a reference profile of the quantity of septin family protein expression or fragments thereof and the quantity of CA125 and/or HE4, said reference profile representing a known quantity of septin family protein expression or fragments thereof and a known quantity of CA125 and/or HE4 in a sample from the subject pre-surgery; (iv) finding a deviation or no deviation of the subject profile of (ii) from the reference profile; and (v) attributing said finding of deviation or no deviation to a particular prognosis for Müllerian cancer in said subject. In other embodiments, the prognosis for the subject is classified as poor based on a deviation of any of 5%, 10%, 15%, 20%, 25%, 30%, 35%, 40%, 45%, 50%, 55%, 60%, 65%, 70%, 75%, 80%, 85%, 90%, 95%, 100%, or more (inclusive of values falling in between these percentages) compared to the reference profile.

Further, a method for monitoring the chemotherapy response of Müllerian tumors based on the septin family member expression (such as, but not limited to, septin-2 expression) is provided herein. In this embodiment, the septin family member expression levels in subject samples can be indicative of the disease burden, recurrence, progression, regression, prognostic indicator, survival rate, or otherwise can indicate systemic toxicity that can disable the subject.

Alternatively, in other embodiments, the measured level(s) of biomarker(s) (such as septin family members, for example, septin-2) are not compared to a reference value, but are used in an equation, wherein each biomarker level is given a certain weight. This leads to a numerical value, which can be projected on a risk scale. Said scale then indicates the ranges of the numerical values pointing towards 1) benign, or 2) malignant Müllerian tumors, preferably pointing towards 1) benign and 2) early malignant, or borderline (LMP) tumors. Using the same equation, the numerical value obtained from the analysis of a sample of the subject can then be projected on the reference scale in order to predict the risk of having or developing a Müllerian cancer. The quantities, measurements or scores for the biomarker(s) and parameter(s) may thus each be modulated by an appropriate weighing factor and added up to yield a single value, which can then be suitably compared with a corresponding reference value obtained accordingly.

### D. Anti-cancer therapies

The methods of the disclosed herein may be practiced in an adjuvant setting. "Adjuvant setting" refers to a clinical setting in which a subject has had a history of a proliferative disease, particularly a Müllerian cancer (such as ovarian cancer), and generally (but not necessarily) has been responsive to therapy, which includes, but is not limited to, surgery, radiotherapy, and chemotherapy. However, because of a history of the proliferative disease (such as a Müllerian cancer, for example, ovarian cancer), these subjects are considered at risk of developing that disease. Treatment or administration in the "adjuvant setting" refers to a subsequent mode of treatment.

The methods provided herein may also be practiced in a "neoadjuvant setting," that is, the method may be carried out before the primary/definitive therapy. In some aspects, the subject has previously been treated. In other aspects, the subject has not previously been treated. In some aspects, the treatment is a first line therapy. The subject may be a human or may be a non-human mammal.

In some aspects, any of the methods described herein include the administration of an effective amount of an anti-cancer therapy (such as a therapy that inhibits the expression of a septin family gene or protein or fragment thereof, e.g., septin-2) to the tumors of subject having or suspected of having a Müllerian cancer.

For prophylactic use, beneficial or desired results include results such as eliminating or reducing the risk, lessening the severity, or delaying the onset of Müllerian carcinogenesis (such as ovarian carcinogenesis), including biochemical, histological and/or behavioral symptoms of Müllerian cancer, its complications and intermediate pathological phenotypes presenting during development of Müllerian cancer. For therapeutic use, beneficial or desired results include clinical results such as decreasing one or more symptoms resulting from Müllerian cancer, increasing the quality of life of those suffering from Müllerian cancer, decreasing the dose of other medications required to treat the Müllerian cancer, enhancing effect of another medication such as via targeting, delaying the progression of the disease, and/or prolonging survival. An effective dosage can be administered in one or more administrations. For purposes of this disclosure, an effective dosage of an anti-cancer therapy is an amount sufficient to accomplish prophylactic or therapeutic treatment either directly or indirectly. As is understood in the clinical context, an effective dosage of an anti-cancer therapy may or may not be achieved in conjunction with another anti-cancer therapy.

The therapeutic methods disclosed herein encompass inhibiting the expression or activity of a septin family member gene, protein, or fragment thereof for the treatment of a proliferative disease in a subject in need thereof.

### 1. Antibodies

The therapeutic methods disclosed herein can comprise inhibiting the expression or activity of a septin family member protein, or fragment thereof, by administering one or more antibodies directed to one or more septin family member proteins (such as, septin-2). "Antibody" as used herein is meant to include intact molecules as well as fragments which retain the ability to bind antigen (*e.g.,* Fab and F(ab') fragments). These fragments are typically produced by proteolytically cleaving intact antibodies using enzymes such as a papain (to produce Fab fragments) or pepsin (to produce F(ab')2 fragments). The term "antibody" also refers to both monoclonal antibodies and polyclonal antibodies. Polyclonal antibodies are derived from the sera of animals immunized with the antigen. Monoclonal antibodies can be prepared using hybridoma technology (Kohler, et al., Nature 256:495 (1975)). In general, this technology involves immunizing an animal, usually a mouse, with the CA125 peptide. The splenocytes of the immunized animals are extracted and fused with suitable myeloma cells, *e.g.,* SP2O cells. After fusion, the resulting hybridoma cells are selectively maintained in a culture medium and then cloned by limiting dilution (Wands, et al., Gastroenterology 80:225-232 (1981)). The cells obtained through such selection are then assayed to identify clones which secrete antibodies capable of binding to septin family member proteins or fragments thereof.

### 2. Non-antibody binding polypeptides

The therapeutic methods disclosed herein can comprise inhibiting the activity expression of a septin family member protein, or fragment thereof, by administering one or more non-antibody binding polypeptide directed to one or more septin family member proteins (such as, septin-2). Binding polypeptides are polypeptides that bind, preferably specifically, to one or more septin family member proteins or fragments thereof). Binding polypeptides may be chemically synthesized using known polypeptide synthesis methodology or may be prepared and purified using recombinant technology. Binding polypeptides are usually at least about 5 amino acids in length, alternatively at least about 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, or 100 amino acids in length or more, wherein such binding polypeptides that are capable of binding, preferably specifically, to septin family member proteins or a fragment thereof. Binding polypeptides may be identified without undue experimentation using well known techniques. In this regard, it is noted that techniques for screening polypeptide libraries for binding polypeptides that are capable of binding to a polypeptide target are well known in the art (*see, e.g.,* U.S. Pat. Nos. 5,556,762, 5,750,373, 4,708,871, 4,833,092, 5,223,409, 5,403,484, 5,571,689, 5,663,143; PCT Publication Nos. WO 84/03506 and WO84/03564; Cwirla, S. E. et al., (1990) Proc. Natl. Acad. Sci. USA, 87:6378; Lowman, H. B. et al., (1991) Biochemistry, 30:10832; Clackson, T. et al., (1991) Nature, 352: 624; Marks, J. D. et al., (1991), J. Mol. Biol., 222:581; Kang, A. S. et al., (1991) Proc. Natl. Acad. Sci. USA, 88:8363, and Smith, G. P. (1991) Current Opin. Biotechnol., 2:668).

### 3. Small molecule chemical compounds

The therapeutic methods disclosed herein can comprise inhibiting the expression or activity of a septin family member gene, protein, or fragment thereof, by administering one or more small molecule chemical compounds directed to one or more septin family member proteins (such as, septin-2). The small molecule chemical compound may be a component of a combinatorial chemical library. Combinatorial chemical libraries are a collection of multiple species of chemical compounds comprised of smaller subunits or monomers. Combinatorial libraries come in a variety of sizes, ranging from a few hundred to many hundreds of thousand different species of chemical compounds. There are also a variety of library types, including oligomeric and polymeric libraries comprised of compounds such as carbohydrates, oligonucleotides, and small organic molecules, etc. Such libraries have a variety of uses, such as immobilization and chromatographic separation of chemical compounds, as well as uses for identifying and characterizing ligands capable of binding an acceptor molecule (such as a septin family member gene, protein, or fragment thereof) or mediating a biological activity of interest (such as, but not limited to, inhibition of cellular proliferation).

Various techniques for synthesizing libraries of compounds on solid-phase supports are known in the art. Solid-phase supports are typically polymeric objects with surfaces that are functionalized to bind with subunits or monomers to form the compounds of the library. Synthesis of one library typically involves a large number of solid-phase supports. To make a combinatorial library, solid-phase supports are reacted with one or more subunits of the compounds and with one or more numbers of reagents in a carefully controlled, predetermined sequence of chemical reactions. In other words, the library subunits are "grown" on the solid-phase supports. The larger the library, the greater the number of reactions required, complicating the task of keeping track of the chemical composition of the multiple species of compounds that make up the library.

Small molecules may be identified and chemically synthesized using known methodology (see, e.g., International Patent Application Publication Nos. WO00/00823 and WO00/39585). Small molecules are usually less than about 2000 Daltons in size or alternatively less than about 1500, 750, 500, 250 or 200 Daltons in size, wherein such small molecules that are capable of binding, preferably specifically, to a septin family member gene, protein, or fragment thereof (such as septin-2) as described herein may be identified without undue experimentation using well known techniques. In this regard, it is noted that techniques for screening small molecule libraries for molecules that are capable of binding to a polypeptide target are well known in the art (see, e.g., PCT Publication Nos. WO00/00823 and WO00/39585). Small molecules may be, for example, aldehydes, ketones, oximes, hydrazones, semicarbazones, carbazides, primary amines, secondary amines, tertiary amines, N-substituted hydrazines, hydrazides, alcohols, ethers, thiols, thioethers, disulfides, carboxylic acids, esters, amides, ureas, carbamates, carbonates, ketals, thioketals, acetals, thioacetals, aryl halides, aryl sulfonates, alkyl halides, alkyl sulfonates, aromatic compounds, heterocyclic compounds, anilines, alkenes, alkynes, diols, amino alcohols, oxazolidines, oxazolines, thiazolidines, thiazolines, enamines, sulfonamides, epoxides, aziridines, isocyanates, sulfonyl chlorides, diazo compounds, acid chlorides, or the like.

### 4. Nucleic acids

The therapeutic methods disclosed herein can comprise inhibiting the expression of a septin family member gene, protein, or fragment thereof, by administering one or more nucleic acids directed to one or more septin family member DNA or RNA (such as, septin-2). Such nucleic acids can include, without limitations, antisense oligonucleotoides, small inhibitory RNAs (siRNAs), triplex-forming oligonucleotides, ribozymes, or any other inhibitory oligonucleotide or nucleic acid. In addition, the nucleic acid-based therapeutics for use in the methods described herein can have one or more alterations to the oligonucleotide phosphate backbone, sugar moieties, and/or nucleobase (such as any of those described herein) that increase resistance to degradation, such as by nuclease cleavage. Nucleic acids complementary to one or more septin family genes or RNAs (such as septin-2) are at least about 10 (such as any of about 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50) nucleotides in length. In another embodiment, the nucleic acids can be between about 10-15, 15-20, 20-25, 25-30, 30-35, 35-40, 40-45, or 45-50 oligonucleotides in length.

The naturally occurring internucleoside linkage of RNA and DNA is a 3' to 5 phosphodiester linkage. The nucleic acids used according to any of the methods disclosed herein can have one or more modified, i.e. non-naturally occurring, internucleoside linkages. With respect to therapeutics, modified internucleoside linkages are often selected over oligonucleotides having naturally occurring internucleoside linkages because of desirable properties such as, for example, enhanced cellular uptake, enhanced affinity for target nucleic acids, and increased stability in the presence of nucleases.

Oligonucleotides (such as an antisense oligonucleotide) having modified internucleoside linkages include internucleoside linkages that retain a phosphorus atom as well as internucleoside linkages that do not have a phosphorus atom. Representative phosphorus containing internucleoside linkages include, but are not limited to, phosphodiesters, phosphotriesters, methylphospho nates, phosphoramidate, and phosphorothioates. Methods of preparation of phosphorous-containing and non-phosphorous-containing linkages are well known.

As is known in the art, a nucleoside is a base-sugar combination. The base portion of the nucleoside is normally a heterocyclic base. The two most common classes of such heterocyclic bases are the purines and the pyrimidines. Nucleotides are nucleosides that further include a phosphate group covalently linked to the sugar portion of the nucleoside. For those nucleosides that include a pentofuranosyl sugar, the phosphate group can be linked to either the 2', 3' or 5' hydroxyl moiety of the sugar. In forming oligonucleotides, the phosphate groups covalently link adjacent nucleosides to one another to form a linear polymeric compound. In turn the respective ends of this linear polymeric structure can be further joined to form a circular structure, however, open linear structures are generally preferred. Within the oligonucleotide structure, the phosphate groups are commonly referred to as forming the internucleoside backbone of the oligonucleotide. The normal linkage or backbone of RNA and DNA is a 3' to 5' phosphodiester linkage.

Specific though nonlimiting examples of nucleic acids (such as antisense oligonucleotides) useful in the methods of the present invention include oligonucleotides containing modified backbones or non-natural internucleoside linkages. As defined in this specification, oligonucleotides having modified backbones include those that retain a phosphorus atom in the backbone and those that do not have a phosphorus atom in the backbone. For the purposes of this specification, and as sometimes referenced in the art, modified oligonucleotides that do not have a phosphorus atom in their internucleoside backbone can also be considered to be oligonucleosides.

In some embodiments, modified oligonucleotide backbones include, for example, phosphorothioates, chiral phosphorothioates, phosphorodithioates, phosphotriesters, aminoalkylphosphotri-esters, methyl and other alkyl phosphonates including 3'-alkylene phosphonates, 5'-alkylene phosphonates and chiral phosphonates, phosphinates, phosphoramidates including 3'-amino phosphoramidate and aminoalkylphosphoramidates, thiono-phosphoramidates, thionoalkylphosphonates, thionoalkylphospho-triesters, selenophosphates and boranophosphates having normal 3 '-5' linkages, 2'-5' linked analogs of these, and those having inverted polarity wherein one or more internucleotide linkages is a 3' to 3', 5' to 5' or 2' to 2' linkage. Oligonucleotides having inverted polarity comprise a single 3' to 3' linkage at the 3'- most internucleotide linkage i.e. a single inverted nucleoside residue which may be abasic (the nucleobase is missing or has a hydroxyl group in place thereof) can also be employed. Various salts, mixed salts and free acid forms are also included.

Oligonucleotide backbones that do not include a phosphorus atom therein have backbones that are formed by short chain alkyl or cycloalkyl internucleoside linkages, mixed heteroatom and alkyl or cycloalkyl internucleoside linkages, or one or more short chain heteroatomic or heterocyclic internucleoside linkages. These include those having morpholino linkages (formed in part from the sugar portion of a nucleoside); siloxane backbones; sulfide, sulfoxide and sulfone backbones; formacetyl and thioformacetyl backbones; methylene formacetyl and thioformacetyl backbones; riboacetyl backbones; alkene containing backbones; sulfamate backbones; methyleneimino and methylenehydrazino backbones; sulfonate and sulfonamide backbones; amide backbones; and others having mixed N, O, S and C component parts.

Representative United States patents that teach the preparation of the above phosphorus-containing and non-phosphorus-containing linkages include, but are not limited to, U.S. Pat. Nos. 5,541,306; 5,550,111; 5,563,253; 5,571,799; 5,587,361; 5,194,599; 5,565,555; 5,527,899; 5,721,218; 5,672,697 and 5,625,050, 5,596,086; 5,602,240; 5,610,289; 5,602,240; 5,608,046; 5,610,289; 5,618,704; 5,623,070; 5,663,312; 5,633,360; 5,677,437; 5,792,608; 5,646,269 and 5,677,439.

Modified nucleic acids (such as antisense oligonucleotides) complementary to one or more septin family DNA or RNA sequences used as anticancer therapies in conjunction with any of the methods disclosed herein may also contain one or more substituted or modified sugar moieties. For example, the furanosyl sugar ring can be modified in a number of ways including substitution with a substituent group, bridging to form a bicyclic nucleic acid "BNA" and substitution of the 4'-0 with a heteroatom such as S or N(R) as described in U.S. Pat. No. 7,399,845 Other examples of BNAs are described in published International Patent Application No. WO 2007/146511.

Nucleic acids (such as antisense oligonucleotides) for use in any of the methods disclosed herein may also include nucleobase (often referred to in the art simply as "base") modifications or substitutions. Nucleobase modifications or substitutions are structurally distinguishable from, yet functionally interchangeable with, naturally occurring or synthetic unmodified nucleobases. Both natural and modified nucleobases are capable of participating in hydrogen bonding. Such nucleobase modifications may impart nuclease stability, binding affinity or some other beneficial biological property to oligonucleotide compounds. Modified nucleobases include synthetic and natural nucleobases such as, for example, 5-methylcytosine (5-me-C). Certain nucleobase substitutions, including 5-methylcytosine substitutions, are particularly useful for increasing the binding affinity of an oligonucleotide compound (such as an antisense oligonucleotide compound) for a target nucleic acid (such as an septin family member nucleic acid, for example, septin-2).

Additional unmodified nucleobases include 5-hydroxymethyl cytosine, xanthine, hypoxanthine, 2-aminoadenine, 6-methyl and other alkyl derivatives of adenine and guanine, 2-propyl and other alkyl derivatives of adenine and guanine, 2-thiouracil, 2-thiothymine and 2-thiocytosine, 5-halouracil and cytosine, 5-propynyl (-C=C-CH3) uracil and cytosine and other alkynyl derivatives of pyrimidine bases, 6-azo uracil, cytosine and thymine, 5-uracil (pseudouracil), 4-thiouracil, 8-halo, 8-amino, 8-thiol, 8-thioalkyl, 8-hydroxyl and other 8- substituted adenines and guanines, 5-halo particularly 5-bromo, 5-trifluoromethyl and other 5-substituted uracils and cytosines, 7-methylguanine and 7-methyladenine, 2-F-adenine, 2- amino-adenine, 8-azaguanine and 8-azaadenine, 7-deazaguanine and 7-deazaadenine and 3- deazaguanine and 3-deazaadenine.

Heterocyclic base moieties may also include those in which the purine or pyrimidine base is replaced with other heterocycles, for example 7-deaza-adenine, 7-deazaguanosine, 2- aminopyridine and 2-pyridone. Nucleobases that are particularly useful for increasing the binding affinity of antisense compounds include 5-substituted pyrimidines, 6-azapyrimidines and N-2, N-6 and 0-6 substituted purines, including 2 aminopropyladenine, 5-propynyluracil and 5-propynylcytosine.

As used herein, "unmodified" or "natural" nucleobases include the purine bases adenine (A) and guanine (G), and the pyrimidine bases thymine (T), cytosine (C) and uracil (U).

### 5. Pharmaceutical compositions

Any of the anticancer therapies (such as oligonucleotide-based therapies) disclosed herein can be administered in the form of pharmaceutical compositions. These compounds can be administered by a variety of routes including oral, rectal, transdermal, subcutaneous, intravenous, intramuscular, and intranasal. These compounds are effective as both injectable and oral compositions. Such compositions are prepared in a manner well known in the pharmaceutical art and comprise at least one active compound. When employed as oral compositions, the oligonucleotides and another disclosed herein are protected from acid digestion in the stomach by a pharmaceutically acceptable protectant.

This disclosure also includes pharmaceutical compositions which contain, as the active ingredient, one or more of the anticancer therapies disclosed herein associated with one or more pharmaceutically acceptable excipients or carriers. In making the compositions of this disclosure, the active ingredient is usually mixed with an excipient or carrier, diluted by an excipient or carrier or enclosed within such an excipient or carrier which can be in the form of a capsule, sachet, paper or other container. When the excipient or carrier serves as a diluent, it can be a solid, semi-solid, or liquid material, which acts as a vehicle, carrier or medium for the active ingredient. Thus, the compositions can be in the form of tablets, pills, powders, lozenges, sachets, cachets, elixirs, suspensions, emulsions, solutions, syrups, aerosols (as a solid or in a liquid medium), ointments containing, for example, up to 10% by weight of the active compound, soft and hard gelatin capsules, suppositories, sterile injectable solutions, and sterile packaged powders.

In preparing a formulation, it may be necessary to mill the active lyophilized compound to provide the appropriate particle size prior to combining with the other ingredients. If the active compound is substantially insoluble, it ordinarily is milled to a particle size of less than 200 mesh. If the active compound is substantially water soluble, the particle size is normally adjusted by milling to provide a substantially uniform distribution in the formulation, e.g. about 40 mesh.

Some examples of suitable excipients or carriers include lactose, dextrose, sucrose, sorbitol, mannitol, starches, gum acacia, calcium phosphate, alginates, tragacanth, gelatin, calcium silicate, microcrystalline cellulose, polyvinylpyrrolidone, cellulose, sterile water, syrup, and methyl cellulose. The formulations can additionally include: lubricating agents such as talc, magnesium stearate, and mineral oil; wetting agents; emulsifying and suspending agents; preserving agents such as methyl- and propylhydroxy-benzoates; sweetening agents; and flavoring agents. The compositions of the disclosure can be formulated so as to provide quick, sustained or delayed release of the active ingredient after administration to the subject by employing procedures known in the art.

The compositions can be formulated in a unit dosage form, each dosage containing from about 5 mg to about 100 mg or more, such as any of about 1 mg to about 5 mg, 1 mg to about 10 mg, about 1 mg to about 20 mg, about 1 mg to about 30 mg, about 1 mg to about 40 mg, about 1 mg to about 50 mg, about 1 mg to about 60 mg, about 1 mg to about 70 mg, about 1 mg to about 80 mg, or about 1 mg to about 90 mg, inclusive, including any range in between these values, of the active ingredient. The term "unit dosage forms" refers to physically discrete units suitable as unitary dosages for subjects, each unit containing a predetermined quantity of active material calculated to produce the desired therapeutic effect, in association with a suitable pharmaceutical excipient or carrier.

The anticancer therapies disclosed herein are effective over a wide dosage range and are generally administered in a therapeutically effective amount. It will be understood, however, that the amount of the anticancer therapies actually administered will be determined by a physician, in the light of the relevant circumstances, including the condition to be treated, the chosen route of administration, the actual compound administered, the age, weight, and response of the individual subject, the severity of the subject's symptoms, and the like.

The tablets or pills can be coated or otherwise compounded to provide a dosage form affording the advantage of prolonged action and to protect the anticancer therapies (such as an oligonucleotide) from acid hydrolysis in the stomach. For example, the tablet or pill can comprise an inner dosage and an outer dosage component, the latter being in the form of an envelope over the former. The two components can be separated by an enteric layer which serves to resist disintegration in the stomach and permit the inner component to pass intact into the duodenum or to be delayed in release. A variety of materials can be used for such enteric layers or coatings, such materials including a number of polymeric acids and mixtures of polymeric acids with such materials as shellac, cetyl alcohol, and cellulose acetate.

The liquid forms in which the novel compositions of the present disclosure can be incorporated for administration orally or by injection include aqueous solutions, suitably flavored syrups, aqueous or oil suspensions, and flavored emulsions with edible oils such as corn oil, cottonseed oil, sesame oil, coconut oil, or peanut oil, as well as elixirs and similar pharmaceutical vehicles.

Compositions for inhalation or insufflation include solutions and suspensions in pharmaceutically acceptable, aqueous or organic solvents, or mixtures thereof, and powders. The liquid or solid compositions can contain suitable pharmaceutically acceptable excipients as described herein. The compositions can be administered by the oral or nasal respiratory route for local or systemic effect. Compositions in pharmaceutically acceptable solvents can be nebulized by use of inert gases. Nebulized solutions can be inhaled directly from the nebulizing device or the nebulizing device can be attached to a face mask tent, or intermittent positive pressure breathing machine. Solution, suspension, or powder compositions can also be administered, orally or nasally, from devices which deliver the formulation in an appropriate manner.

### 6. Other anti-cancer therapies

In some aspects, any of the methods of treatment described herein can further comprise administering one or more additional anti-cancer therapies to the subject. Various classes of anti-cancer agents can be used. Non-limiting examples include: alkylating agents, antimetabolites, anthracyclines, plant alkaloids, topoisomerase inhibitors, podophyllotoxin, antibodies (*e.g.,* monoclonal or polyclonal), tyrosine kinase inhibitors (*e.g.,* imatinib mesylate (Gleevec® or Glivec®)), hormone treatments, soluble receptors and other antineoplastics.

Topoisomerase inhibitors are also another class of anti-cancer agents that can be used. Topoisomerases are essential enzymes that maintain the topology of DNA. Inhibition of type I or type II topoisomerases interferes with both transcription and replication of DNA by upsetting proper DNA supercoiling. Some type I topoisomerase inhibitors include camptothecins irinotecan and topotecan. Examples of type II inhibitors include amsacrine, etoposide, etoposide phosphate, and teniposide. These are semisynthetic derivatives of epipodophyllotoxins, alkaloids naturally occurring in the root of American Mayapple (Podophyllum peltatum).

Antineoplastics include the immunosuppressant dactinomycin, doxorubicin, epirubicin, bleomycin, mechlorethamine, cyclophosphamide, chlorambucil, ifosfamide. The antineoplastic compounds generally work by chemically modifying a cell's DNA.

Alkylating agents can alkylate many nucleophilic functional groups under conditions present in cells. Cisplatin and carboplatin, and oxaliplatin are alkylating agents. They impair cell function by forming covalent bonds with the amino, carboxyl, sulfhydryl, and phosphate groups in biologically important molecules.

Vinca alkaloids bind to specific sites on tubulin, inhibiting the assembly of tubulin into microtubules (M phase of the cell cycle). The vinca alkaloids include: vincristine, vinblastine, vinorelbine, and vindesine.

Anti-metabolites resemble purines (azathioprine, mercaptopurine) or pyrimidine and prevent these substances from becoming incorporated in to DNA during the "S" phase of the cell cycle, stopping normal development and division. Anti-metabolites also affect RNA synthesis.

Plant alkaloids and terpenoids are derived from plants and block cell division by preventing microtubule function. Since microtubules are vital for cell division, without them, cell division cannot occur. The main examples are vinca alkaloids and taxanes.

Podophyllotoxin is a plant-derived compound which has been reported to help with digestion as well as used to produce two other cytostatic drugs, etoposide and teniposide. They prevent the cell from entering the Gl phase (the start of DNA replication) and the replication of DNA (the S phase).

Taxanes as a group includes paclitaxel and docetaxel. Paclitaxel is a natural product, originally known as Taxol and first derived from the bark of the Pacific Yew tree. Docetaxel is a semi- synthetic analogue of paclitaxel. Taxanes enhance stability of microtubules, preventing the separation of chromosomes during anaphase.

The methods and agents derived from this disclosure may be administered in combination with other therapies such as, for example, radiation therapy, surgery, conventional chemotherapy or with a combination of one or more additional therapies. The methods and agents derived from this disclosure may be administered alone in a pharmaceutical composition or combined with therapeutically effective and physiologically acceptable amount of one or more other active ingredients or agents. Such other active ingredient includes, but is not limited to glutathione antagonists, angiogenesis inhibitors, chemotherapeutic agent(s) and antibodies (*e.g*., cancer antibodies). The agents described in this disclosure may be administered simultaneously or sequentially. The separation in time between administrations may be minutes, hours, days or it may be longer.

For example, inhibitors of one or more septin family genes, proteins, or fragment thereof (such as, septin-2) activity or expression can be administered before, after, or simultaneously with chemotherapeutic and/or cytotoxic agents such as alkylating agents (*e.g.* , chlorambucil, cyclophosphamide, ccnu, melphalan, procarbazine, thiotepa, bcnu, and busulfan), antimetabolites (*e.g.* , 6- mercaptopurine and 5-fluorouracil), anthracyclines (*e.g.* , daunorubicin, doxorubicin, idarubicin, epirubicin, and mitoxantrone), antitumor antibiotics (*e.g.,* bleomycin), monoclonal antibodies (*e.g.* , alemtuzumab, bevacizumab, cetuximab, gemtuzumab, ibritumomab, panitumumab, rituximab, tositumomab, and trastuzumab), platinums (*e.g.* , cisplatin, oxaliplatin, and carboplatin), plant alkaloids (*e.g.,* vincristine), topoisomerase I or II inhibitors (*e.g*., irinotecan, topotecan, amsacrine, etoposide, etoposide phosphate, and teniposide), vinca alkaloids (*e.g.* , vincristine, vinblastine, vinorelbine, and vindesine), taxanes (*e.g.,* paclitaxel and docetaxel), epipodophyllotoxins (*e.g.,* etoposide and teniposide), nucleoside analogs, and angiogenesis inhibitors (*e.g*., Avastin (beracizumab), a humanized monoclonal antibody specific for VEGF-A).

Examples of glutathione antagonists include but are not limited to buthionine sulfoximine, cyclophosphamide, ifosphamide, actinomycin-d and N-(4-hydroxyphenyl) retinamide (4-HPR). Examples of angiogenesis inhibitors include but are not limited to 2-methoxyestradiol(2-ME), AG3340, Angiostatin, antithrombin-III, Anti-VEGF antibody, Batimastat, bevacizumab (Avastin), BMS-275291 , CA1 , Canstatin, combretastatin, Combretastatin-A4 phosphate, CC-5013, captopril, celecoxib, Dalteparin, EMD121974, Endostatin, Erlotinib, Gefitinib, Genistein, Halofuginone, ID 1 , ID3, IM862, Imatinib mesylate, Inducible protein- 10, Interferon-alpha, Interleukin-12, Lavendustin-a, LY317615, or AE-941 , Marimastat, Mapsin, Medroxyprogesterone acetate, Meth-1, Meth-2, Neovastat, Osteopontin cleaved product, PEX, Pigment epithelium growth factor (PEGF), platelet growth factor 4, prolactin fragment, proliferin-related protein(PRP), PTK787/ZK222584, recombinant human platelet factor-4(rPF4), restin, squalamine, SU5416, SU6668, Suramin, Taxol, Tecogalan, Thalidomide, Tetrathiomolybdate (TM), Thrombospondin, TNP-470, Troponin I, Vasostatin, VEGF1 , VEGF-TPvAP and ZD6474. In some embodiment the angiogenesis inhibitor is a VRGF antagonist. The VEGF antagonist may be a VEGF binding molecule. VEGF binding molecule include VEGF antibodies, or antigen binding fragment (s) thereof. One example of a VEGF antagonist is NeXstar.

Examples of categories of chemotherapeutic agents that may be used in any of the methods or agents disclosed herein include, but are not limited to, DNA damaging agents and these include topoisomerase inhibitors (*e.g*. , etoposide, camptothecin, topotecan, irinotecan, teniposide, mitoxantrone), anti -microtubule agents (*e.g.* , vincristine,vinblastine), antimetabolite agents (*e.g.* , cytarabine, methotrexate, hydroxyurea, 5- fluorouracil, flouridine, 6-thioguanine, 6-mercaptompurine, fludarabine, pentostatin, chlorodeoxyadenosine), DNA alkylating agents (*e.g.,* cisplatin, mecholorethamine, cyclophosphamide, ifosphamide, melphalan, chlorambucil, busulfan, thiotepa, carmustine, lomustine, carboplatin, dacarbazine, procarbazine) and DNA strand break inducing agents( *e.g.* , bleomycin, doxorubicin, daunorubicin, idarubicin, mitomycin C).

Chemotherapeutic agents include synthetic, semisynthetic and naturally derived agents. Important chemotherapeutic agents include, but are not limited to, Avicine, Aclarubicin, Acodazole, Acronine, Adozelesin, Adriamycin, aldesleukin, Alitretinoin, AUopurinol sodium, Altretamine, Ambomycin, Ametantrone acetate, Aminoglutethimide, Amsacrine, Anastrazole, Annonaceous Acetogenins, Anthramycin, Asimicin, Asparaginase, asperlin, Azacitidine, azetepa, Azotomycin, batimastat, benzodepa, bexarotene, Bicalutamide, Bisantrene, Bisnafide, Bizelesin, Bleomycin, Brequinar, Bropirimine, Bullatacin, Busulfan, Cabergoline, cactinomycin, calusterone, caracemide, carbetimer, carboplatin, carmustine, carubicin, carzelesin, cedefingol, chlorambucil, celecoxib, cirolemycin, cisplatin, cladribine, crisnatol, cyclophosphamide, cytarabine, dacarbazine, DACA, dactinomycin, Daunorubicin, daunomycin, Decitabine, denileukin, Dexormaplatin, Dezaguanine, Diaziquone, Docetaxel, Doxorubicin, Droloxifene, Dromostalone, Duazomycin, Edatrexate, Eflornithine, Elsamitrucin, Estramustine, Etanidazole, Etoposide, Etoprine, Fadrozole, Fazarabine, Fenretinide, Floxuridine, Fludarabine, Fluorouracil, Flurocitabine, 5-FdUMP, Fosquidone, Fosteuecine, FK-317, FK-973, FR-66979, FR-900482, Gemcitabine, Gemtuzumab, Ozogamicin, Gold Aul 98, Goserelin, Guanacone, Hydroxyurea, Idarubicin, Ilmofosine, Interferon alpha and analogs, Iproplatin, irinotecan, Lanreotide, Letrozole, Leuprolide, Liarozole, Lometrexol, Lomustine, Losoxantrone, masoprocol, Maytansine, Mechlorethamine, Megestrol, Melengestrol, Melphalan, Menogaril, Metoprine, maturedepa, mitindomide, Mitocarcin, Mitogillin, Mitomalacin, Mitomycin, Mitomycin C, Mitosper, Mitotane, Mitoxantrone, Mycophenolic acid, Nocodazole, Nogalamycin, Oprelvekin, ormaplatin, Oxisuran, Paclitaxel, pamidronate, pegaspargase, Peliomycin, Pentamustine, Peplomycin, Perfosfamide, Pipobroman, Piposulfan, Piroxantrone, Plicamycin, Plomestane, Porfimer, Porfiromycin, Prednimustine, procarbazine, Puromycin, Pyrazofurin, Riboprine, Rituximab, Rogletimide, Rolliniastatin, safingol, Samarium, Semustine, Simtrazene, Sparfosate, Sparsomycin, spirogermanium, Spiromustine, Spiroplatin, Squamocin, Squamotacin, streptonigrin, streptozocin, SrC12, Sulphofenur, Talisomycin, Taxane, Toxoid, Tecoglan, Tegafur, teloxantrone, Temoporfin, teniposide, Teroxirone, Testolactone, Thiamiprine, Thiotepa, Thymitaq, Tiazofurin, Tirapazamine, Tomudex, Top-53, Topotecan, Toremixifme, Trastuzumab, Trestolone, triciribine, Triciribine, Trimetrexate, trimetrexate glucuronate, Triptorelin, Tubulozole, uracil mustard, Uredepa, valrubicin, vapreotide, Vinblastine, Vincristine, Vindesine, Vinepidine, Vinglycinate, Vinleurosine, Vinorelbine, Vinrosidine, Vinzolidine, Vorozole, Zeniplatin, Zinostatin, Zorubicin, 2-cholrodeoxyrubicine, 2'-deoxyformycin, 9-aminocamptothecin, raltitrexed, N-propargyl-5,8-didezafolic acid, 2-cholo-2'arabinofluoro-2' deoxyadenosine, 2-cholo- 2'-deoxyadenosine, anisomycin, Trichostatin, hPRL-G129R, CEP-751, Linomide, Sulfur mustard, nitrogen mustard, N-methyl-N-nitrosourea, fotemustine, Streptozotocin, dacarbazine, mitozolomide, temozolomide, AZQ, ormaplatin, CI-973, DWA21 14R, JM216, JM335, Bisplatinum, Tomudex, azacitidine, cytrabincine, gemcitabine, 6- mercaptopurine, Hypoxanthine, Teniposide, CPT-1 1 , Doxorubicin, Daunorubicin, Epirubicin, darubicin, losoxantrone, amsacrine, pyrazoloacridine, all trans retinol, 14- hydroxy-retro-retinol, all-trans retinoic acid, N-(4-hydroxyphenyl) retinamide, 13- cisretinoic acid, 3 -methyl TTNEB, 9-cisretenoic acid, fludarabine, and 2-Cda.

Other chemotherapeutic agent include: 20-epil,25-dihydroxyvitamin-D3, 5-ethynyl uracil, abiraterone, aclarubicin, acylfulvene, adecylpenol, adozelesin, aldesleukin, ALL-TK antagonists, altretamine, ambumastine, amidox, amifostine, amino levulinic acid, anagrelide, anastrozole, andrographolide, angiogenesis inhibitors, antagonist D, antagonists D, antarelix, anti-dorsalizing morphogenetic protein-1 , antiandrogen, antiestrogen, antineoplastone, antisense oligonucleotides, aphidicolin, apoptosis gene modulators, apoptosis regulators, apurinic acid, ara-cdp-dl-PTBA, arginine aminase, asulacrine, atamestine, atrimustine, axinamastine 1 and axinamastine 2, axinamastine 3, azasetron, azatoxin, azatyrosine, baccatin III derivatives, balanol, BCR/ABL antagonist, benzochlorins, benzoylsaurosporine, beta lactam derivatives, beta-alethine.

Perillyl alcohol, phenozenomyein, phenyl acetate, phosphatase inhibitors, picibanil, pilocarbine and salts or analogs thereof, pirarubucin, piritrexim, placetin A, placetin B, plasminogen activator inhibitor, platinum complex, phenyl ethyl isothiocyanate and analogs thereof, platinum compounds, platinum triamine complex, podophylotoxin, porfimer sodium, porphyromycin, propyl bis acridones, prostaglnadins J2, protease inhibitors, protein A based immune modulators, PKC inhibitors, microalgal, protein tyrosine phosphatase inhibitors, purine neucleoside phosphorylase inhibitors, purpurins, pyrazoloacridines, pyridoxylated haemoglobn polyoxyethylene conjugate, raf antagonists, raltitrexed, ramosetron, ras farnesyl protein tranaferase inhibitors, rasinhibitors, ras-GAP inhibitors, ratellitptine demethylated, Rhenium Re 186 etidronate, rhizoxine, ribozyme, RII retinide, rogletimide, rosagliatazone and analogs and derivatives thereof, rohitukine, romurtide, roquinimex, rubiginone Bl, ruboxyl, safingol, saintopin, SarCNU, sarcophytol A, sargrmostim, sdi 1 mimetics, semustine, senescence derived inhibitor 1 , sense oligonucleotide, signal transduction inhibitors, signal transduction modulators, single chain antigen binding protein, sizofiran, sobuzoxane, sodium borocaptate, sodium phenyl acetate, solverol, somatomedin binding protein, sonermin, sparfosic acid, spicamycin D, spiromustin, splenopentine, spongistatin 1 , squalamine, stem cell inhibitor, stem cell division inhibitor, stipiamide, stromelysin, sulfinosine, superactive vasoactive intestinal peptide antagonists, suradista, siramin, swainsonine, synthetic glycosaminoglycans, tallimustine, tamoxifen methiodide, tauromustine, tazarotene, tacogalan sodium, tegafur, tellurapyrilium, telomerase inhibitors, temoporfin, tmeozolomide, teniposide, tetrachlorodecaoxide, tetrazomine, thaliblastine, thalidomide, thiocoraline, thrombopoetin and mimetics thereof, thymalfasin, thymopoetin receptor agonist, thymotrinan, thyroid stimulating harmone, tin ethyl etiopurpin, tirapazamine, titanocene and salts thereof, topotecan, topsentin, toremifene, totipotent stem cell factors, translation inhibitors, tretinoin, triacetyluridine, tricribine, trimetrexate, triptorelin, tropisetron, turosteride, tyrosine kinase inhibitors, tyrphostins, UBC inhibitors, ubenimex, urogenital sinus derived growth inhibitory factor, urokinase receptor antagonists, vapreotide, variolin B, vector system, erythrocyte gene therapy, velaresol, veramine, verdins, verteporfin, vinorelbine, vinxaltine, vitaxin, vorozol, zanoterone, zeniplatin, zilascorb and zinostatin.

Other chemotherapeutic agents include: antiproliferative agents (*e.g*., piritrexim isothiocyanate), antiprostatic hypertrophy agents(sitogluside), Benign prostatic hyperplasia therapy agents(*e.g*., tomsulosine, RBX2258), prostate growth inhibitory agents (pentomone) and radioactive agents: Fibrinogen 11 25, fludeoxyglucose F18, Flurodopa F18, Insulin 1125, lobenguane 1123, lodipamide sodium 1131 , lodoantipyrine 1131 , Iodocholesterol 1131 , Iodopyracet 1125, Iofetamine HCL 1123, Iomethin 1131 , Iomethin 1131 , Iothalamate sodium 1125, Iothalamate 1131 , Iotyrosine 1131 , Liothyronine 1125, Merosproprol Hgl 97, Methyl ioodobenzo guanine (MIBG-I131 or MIBGI 123) selenomethionine Se75, Technetium Tc99m furifosmin, technetium Tc99m gluceptate, Tc99m Biscisate, Tc99m disofenin, TC99m gluceptate, Tc99m lidofenin, Tc99m mebrofenin, Tc99m medronate and sodium salts thereof, Tc99m mertiatide, Tc99m oxidronate, Tc99m pentetate and salts thereof, Tc99m sestambi, Tc99m siboroxime, Tc99m succimer, Tc99m sulfur colloid, Tc 99m teboroxime, Tc 99m Tetrofosmin, Tc99m Tiatide, Thyroxine 1125, Thyroxine 1131 , Tolpovidone 1131 , Triolein 1125 and Treoline 1125, and Treoline 131 , MIBG-I123 and MIBG 1131 are especially preferred chemotherapeutic agents for coadministration with the nitrofuran containing pharmaceutical composition of the disclosure.

Another category of chemotherapeutic agents are anticancer supplementary potentiating agents, *e.g.* , antidepressant drugs (Imipramine, desipramine, amitriptyline, clomipramine, trimipramine, doxepin, nortriptyline, protriptyline, amoxapine, and maprotiline), or no-trycyclic anti-depressant drugs (sertraline, trazodone and citalopram), Ca++ antagonists (verapamil, nifedipine, nitrendipine and caroverine), calmodulin inhibitors (prenylamine, trifluoperazine and clomipramine), Amphotericin B, Triparanol analogs (*e.g.* , Tamoxifen), antiarrhythmic drugs (*e.g.* , quinidine), antihypertensive drugs (*e.g.* , reserpine), thiol depleters (*e.g.* , buthionine and sulfoximine) and multiple drug resistance reducing agents such as Cremophor EL.

Other chemotherapeutic agents include: annoceous acetogenins, ascimicin, rolliniastatin, guanocone, squamocin, bullatacin, squamotacin, taxanes, baccatin. One important class of chemotherapeutic agents are taxanes (paclitaxel and docetaxel). The compounds of this disclosure in combination with tamoxifen and aromatase inhibitors arimidex (*e.g*. , anastrazole) are particularly useful for treatment of cancers.

Another important class of molecules that is used to treat cancer in combination with compounds and methods of this disclosure include but are not limited to anti- CD20 mAB, rituximab, Rituxan, Tositumoman, Bexxar, anti-HER2, Trastuzumab, Herceptin, MDX20, antiCA125 mAB, antiHE4 mAB, oregovomab mAB, B43.13 mAB, Ovarex , Breva-REX, AR54, GivaRex, ProstaRex mAB, MDX447, gemtuzumab ozoggamycin, Mylotarg, CMA-676, anti-CD33 mAB, anti-tissue factor protein, Sunol, IOR-C5, C5, anti-EGFR mAB, anti-IFR1R mAB, MDX-447, anti-17-1A mAB, edrecolomab mAB, Panorex, anti-CD20 mAB, (Y-90 lebelled), Ibritumomab Tiuxetan (IDEC-Y2B8), Zevalin, anti-Idiotypic mAB.

In other aspects, provided herein are methods for diagnosing and treating a subject suffering from a Müllerian cancer (such as, without limitation, ovarian cancer, *e.g*.,, clear cell ovarian carcinoma). The method includes or comprises the steps of obtaining a biological sample from the subject; detecting whether a septin family member (such as a septin-2) protein or nucleic acid or fragment thereof is present in the sample; diagnosing the subject with a Müllerian cancer when a septin family protein is present in the sample; and surgically excising tissue from which the biological sample was obtained in the subject. The sample can be, for example, one or more of ovarian, uterine, cervical, fallopian, and/or breast cells or tissue. One (unilateral oophorectomy) or both ovaries (bilateral oophorectomy), the Fallopian tubes (salpingectomy), the uterus (hysterectomy), and/or the omentum (omentectomy) may be removed in the surgical excision step. In additional embodiments, the method also includes administering an effective amount of chemotherapy to the diagnosed subject. The chemotherapy can include administration of one or more of platinum-based drugs (such as, without limitation, carboplatin or cisplatin) and/or a non-platinum based chemotherapy. Non-platinum based chemotherapy appropriate for use in the methods provided herein can include, without limitation, administration of one or more of paclitaxel, topotecan, doxorubicin, epirubicin, gemcitabine, bevacizumab bleomycin, or etoposide. However, in other embodiments, it may be necessary to perform chemotherapy first, followed by surgical excision (*i.e.,* a neoadjuvant treatment).

### E. Detection of Septin family proteins

Also provided herein are complexes that include at least a probe and a septin family (such as a septin-2) protein or nucleic acid or fragment thereof. The septin family protein or nucleic acid present in the complex can be derived from a biological sample obtained from a subject, wherein the sample is, for example, one or more of ovarian, uterine, cervical, fallopian, and/or breast cells or tissue. The probe can be any of the nucleic acids (for example, primers or oligonucleotides) or polypeptides (for example, antibodies (or functional fragments thereof) or non-antibody binding peptides) described herein. Additionally, the biological sample from which the septin family protein or nucleic acid is derived can be processed prior to formation of the complex, such as by sectioning the tissue, fixing the cells or tissue with a chemical fixative, or by isolation of total proteins and/or nucleic acids from the cells or tissue. The complex can be formed by contacting the probe *ex vivo* with a septin family protein or nucleic acid derived from the biological sample or by contacting the probe *ex vivo* with the biological sample itself.

As such, provided herein are complexes comprising (a) a probe and (b) a septin family (such as a septin-2) protein or nucleic acid or fragment thereof, wherein the septin family protein or nucleic acid is derived from a biological sample of ovarian, uterine, cervical, fallopian, or breast cells or tissue. In some embodiments, the probe comprises one or more nucleic acids. The one or more nucleic acids of the probe specifically can, in some embodiments, hybridize to the nucleic acid of SEQ ID NO: 2 or a fragment of SEQ ID NO:2. Moreover, the one or more nucleic acid probes can be primers and an extension reaction (such as, PCR) can be performed subsequent to the complex forming between the primers and the septin family nucleic acid or fragment thereof according to methods that are well known in the art. The nucleic acid probe can additionally be detectably labeled, such as with a fluorescent, enzymatic, colorometric, or radioactive label. In other embodiments, the probe can be an antibody to a septin family member (such as septin-2) or a functional fragment thereof (*e.g.,* a Fab or a F(ab') fragment). The antibody or functional fragment thereof can be a monoclonal antibody or a polyclonal antibody. In some embodiments, the monoclonal antibody or functional fragment thereof can be produced using a recombinantly-expressed septin-2 polypeptide immunogen comprising SEQ ID NO:1 or a fragment of SEQ ID NO:1. In further embodiments, the antibody or fragment thereof can be detectably labeled.

In further aspects, provided herein are methods for detecting a septin family (such as a septin-2) protein or nucleic acid or fragment thereof in a biological sample provided by a subject. The method includes or comprises the steps of: (a) contacting the biological sample with a probe that specifically binds to a septin family protein or nucleic acid or fragment thereof; and (b) (i) detecting the binding between the septin family protein or nucleic acid and the probe; and/ or (ii) detecting the formation of a complex comprising the septin family protein or nucleic acid and the probe. With respect to the biological sample, it can be derived from any tissue prone to developing a Müllerian cancer (such as, without limitation, ovarian, uterine, cervical, fallopian, or breast tissue). The probe can be a nucleic acid and be one or more (such as, 1, 2, 3, 4, 5, 6, 7, 8, 9, 10, 11, 12, 13, 14, 15, 16, 17, 18, 19, 20, 25, 30, 35, 40, 45, 50, or more) nucleotides in length. In some embodiments, the one or more nucleic acid probe(s) specifically hybridize to a nucleic acid of SEQ ID NO: 2 or a fragment of SEQ ID NO:2. The one or more nucleic acids of the probe can additionally be PCR primers and, in this instance, a PCR reaction can be performed subsequent to the complex forming between the PCR primers and the septin family nucleic acid according to methods known in the art. The nucleic acid probe can optionally be detectably labeled, such as with a fluorescent, enzymatic, radioactive, or colorimetric label. In other embodiments, the probe can be an antibody or functional fragment thereof, such as a monoclonal antibody or a polyclonal antibody. The monoclonal antibody can be generated by techniques that are known in the art using a recombinantly-produced septin-2 polypeptide immunogen comprising SEQ ID NO:1 or a fragment of SEQ ID NO:1. The method can further include or comprise the steps of (c) contacting the biological sample with a probe (such as any of the protein or nucleic acid-based probes discussed herein) that specifically binds to one or more polypeptides or nucleic acids selected from the group consisting of HE4, CA125, SMRP, transthyretin, B2-microglobulin, apolipoprotein A-1, prealbumin, transferrin, LPA, YKL-40, FSH, AMH and/or inhibin and (d) detecting the binding between the probe and the one or more polypeptides or nucleic acids selected from the group consisting of plasminogen activator inhibitor 1 (PAI-1), urokinase plasminogen activator (uPA), urokinase plasminogen activator receptor (uPAR), matrix metalloproteinase- 3 (MMP-3) and missing-in-metastasis (MTSS).

### III. Kits and Arrays

Provided herein are kits comprising means for measuring the quantity of septin-2 or other septin family proteins or fragments thereof and/or the quantity of CA125, HE4 and/or one or more other biomarkers. Said means can comprise, respectively, one or more binding agents capable of specifically binding to a septin family protein or fragments thereof, and one or more binding agents capable of specifically binding to said CA125, HE4 and/or one or more other biomarkers. For example, any one of said one or more binding agents may be an antibody, aptamer, photoaptamer, protein, peptide, peptidomimetic or a small molecule. For example, any one of CA125, HE4 and/or one or more other biomarkers may be advantageously immobilized on a solid phase or support. The means for measuring the quantity of a septin family member protein (such as septin-2) or fragments thereof and/or the quantity of CA125, HE4 and/or one or more other biomarkers in the present kits can employ an immunoassay technology or mass spectrometry analysis technology or chromatography technology, or a combination of said technologies.

Also disclosed herein is a kit for the classification of a Müllerian tumor in a subject as taught herein comprising: (a) one or more binding agents capable of specifically binding to a septin family member protein (such as, but not limited to, septin-2) or fragments thereof; (b) preferably, a known quantity or concentration of the septin family member protein or fragments thereof (*e.g.,* for use as controls, standards and/or calibrators); (c) preferably, a reference value of the quantity of septin family proteins or fragments thereof, or means for establishing said reference value. Said components under (a) and/or (c) may be suitably labeled as taught elsewhere in this specification.

Also disclosed is a kit for the classification of a Müllerian tumor in a subject as taught herein comprising: (a) one or more binding agents capable of specifically binding to a septin family protein (such as, but not limited to, septin-2) or fragments thereof; (b) one or more binding agents capable of specifically binding to CA125, HE4 and/or one or more other biomarkers; (c) preferably, a known quantity or concentration of a septin family protein or fragments thereof and a known quantity or concentration of said CA125, HE4, p21 and/or one or more other biomarkers (*e.g*., for use as controls, standards and/or calibrators); (d) preferably, a reference profile of the quantity of a septin family protein or fragments thereof and the quantity of said CA125, HE4 and/or one or more other biomarkers, or means for establishing said reference profiles. Said components under (a), (b) and/or (c) may be suitably labeled as taught elsewhere in this specification.

Also disclosed herein is a binding agent array or microarray comprising: (a) one or more binding agents capable of specifically binding to septin-2 or other septin family proteins or fragments thereof, preferably a known quantity or concentration of said binding agents; and (b) optionally and preferably, one or more binding agents capable of specifically binding to CA125, HE4 and/or one or more other biomarkers, preferably a known quantity or concentration of said binding agents.

It is intended that every maximum numerical limitation given throughout this specification includes every lower numerical limitation, as if such lower numerical limitations were expressly written herein. Every minimum numerical limitation given throughout this specification will include every higher numerical limitation, as if such higher numerical limitations were expressly written herein. Every numerical range given throughout this specification will include every narrower numerical range that falls within such broader numerical range, as if such narrower numerical ranges were all expressly written herein.

The invention can be further understood by reference to the following examples, which are provided by way of illustration and are not meant to be limiting.

### EXAMPLES

### General Techniques

The practice of the present invention will employ, unless otherwise indicated, conventional techniques of molecular biology, microbiology, cell biology, biochemistry, nucleic acid chemistry, and immunology, which are well known to those skilled in the art. Such techniques are explained fully in the literature, such as, Molecular Cloning: A Laboratory Manual, fourth edition (Sambrook et al., 2012) and Molecular Cloning: A Laboratory Manual, third edition (Sambrook and Russel, 2001), (jointly referred to herein as "Sambrook"); Current Protocols in Molecular Biology (F.M. Ausubel et al., eds., 1987, including supplements through 2014); PCR: The Polymerase Chain Reaction, (Mullis et al., eds., 1994); Antibodies: A Laboratory Manual, Second edition, Cold Spring Harbor Laboratory Press, Cold Spring Harbor, NY (Greenfield, ed., 2014), Beaucage et al. eds., Current Protocols in Nucleic Acid Chemistry, John Wiley & Sons, Inc., New York, 2000, (including supplements through 2014) and Gene Transfer and Expression in Mammalian Cells (Makrides, ed., Elsevier Sciences B.V., Amsterdam, 2003).

### Example 1

This example illustrates the relative expression of septin-2 in normal, benign and various phenotypes of ovarian cancer.

### Materials and Methods

IRB approval was received to review the medical records of the paraffin fixed human EOC for HE4 level at the time of surgery, progression free survival, and stage at diagnosis. The data was recorded on a password protected file with no identifying information recorded.

HE4 and Septin-2 expression in paraffin fixed human EOC, benign and normal tissues were examined by microscopy. Tissue sections were deparaffinized and rehydrated with 100, 95 and 70% serial ethanol dilutions. Heat-induced antigen retrieval was then performed using DAKO Antigen Retrieval Solution for 20 min. Tissue sections were blocked with Normal horse blocking serum (Vector Laboratories) for 45 min at room temp before incubating with primary antibodies against HE4 (TA307787, Origene, 1:200) in a humidified chamber overnight at 4°C, followed by secondary Alexafluor green (goat). Slides were carefully washed in TBST buffer and the stained again with Septin-2 (SC20408, Santa Cruz Biotechnology; 1:100) in a humidified chamber overnight at 4°C followed by Dylight 595 secondary antibody (1:1000) that was incubated for 60 min for 1 hour at room temperature in the dark. Benign tissues of either endometrioid or serous histology and normal tissues were stained in similar manner. Imaging was done by a trained independent imaging technician at Rhode Island Hospital. Vectashield media containing DAPI (Vector Laboratories) was used to mount cover-slips for imaging analysis. Sixteen-bit images were acquired with a Nikon E800 microscope (Nikon Inc. NY) using a 40× PlanApo objective. A Spot II digital camera (Diagnostic Instruments, MI) was used to acquire the images. The built-in green filter was used to increase image contrast. Camera settings were based on the brightest slide and subsequent images were acquired keeping the same settings. Image processing and analysis was performed using iVision (BioVision Technologies, version 10.4.11, Exton, PA.) image analysis software. Positive staining was defined through intensity thresholding and integrated optical density (IOD) was calculated by examining the thresholded area multiplied by the mean. All measurements were performed in pixels. Confocal images were acquired with a Nikon C1si confocal (Nikon Inc. NY.) using diode lasers 402, 488 and 561. Serial optical sections were performed with EZ-C1 computer software (Nikon Inc. Mellville, NY). Z series sections were collected at 0.3 µm with a 40× PlanApo lens and a scan zoom of 2. The gain settings were based on the brightest slide and kept constant between specimens. Deconvolution and projections were done in Elements (Nikon Inc. Mellville, NY) computer software.

### Results

Compared to normal and benign tissues, the septin-2 expression was strongly upregulated in ovarian cancer tissues. HE4 low expressing tumors demonstrated lower Septin-2 expression compared to HE4 higher expressors that expressed elevated levels of Septin-2.

As shown in Figs. 1 and 2, Septin-2 expression was highest in serous, regardless of HE4 expression and clear cell and endometroid with high levels of HE4 expression. There exists a clear association of HE4 and Septin-2. Based on this data, it is appears possible to predict the Septin-2 levels by measuring the HE4 levels of vice versa.

### Example 2

This Example illustrates the relative expression of septin-2 in benign and serous ovarian cancer patient derived tissues.

### Materials and Methods

Experimental protocols and reagents are identical to those described in Example 1.

### Results

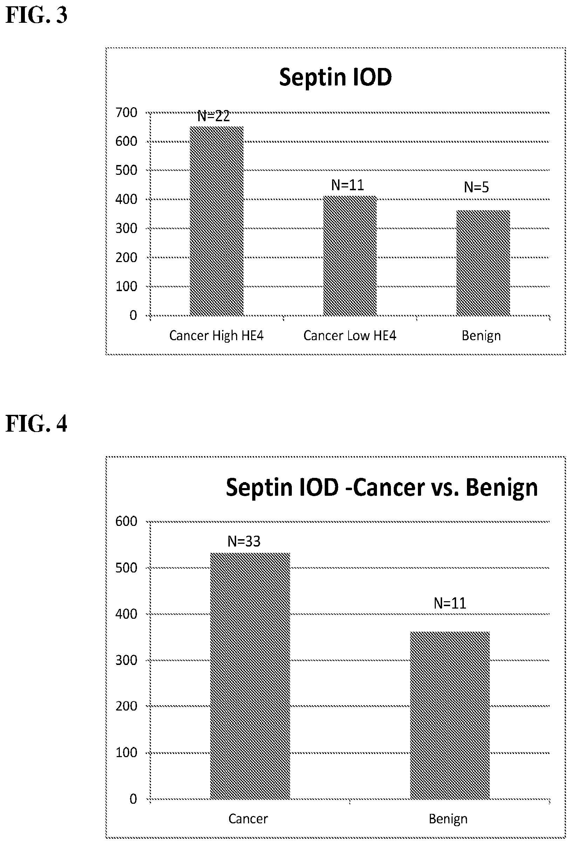

Figure 3 depicts the expression level differences among various phenotypes of epithelial ovarian cancer (EOC) tissues compared to the benign tissues of endometrioid, serous origin. Figure 4 depicts that septin-2 expression in epithelial ovarian cancer (EOC) tissues differ from benign tissues of endometrioid and serous origin. The expression of septin-2 in EOC and benign tissues was measured in terms of Integrated Optical Density (IOD units) as described in Example 1.

### Example 3

This example demonstrates the co-localization of septin-2 and HE4 and describes the relative integrated optical density (IOD) measurements of normal, benign, and ovarian cancer tissue phenotypes.

### Materials and Methods

Experimental protocols and reagents are identical to those described in Example 1.

### Results

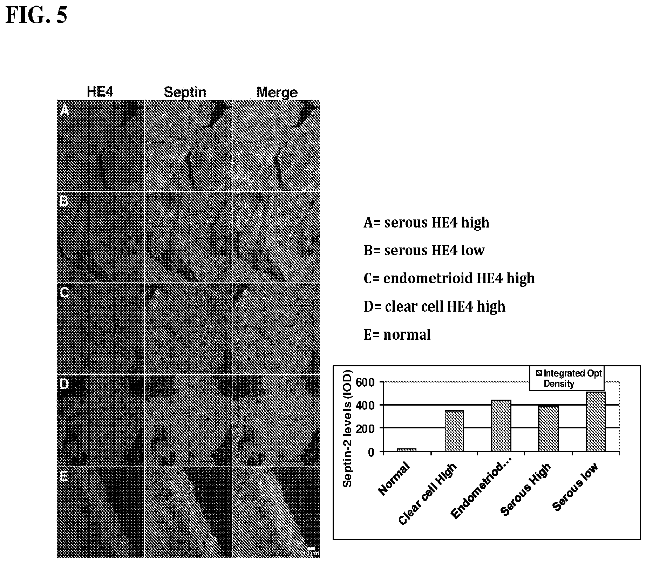

Figure 5 shows co-localization of septin-2 and HE4 in normal, benign and ovarian cancer tissue phenotypes.

### Example 4

This example shows the cytotoxic effects of septin-2 targeting phosphorothio-oligonucleotides (PTOs) in platinum resistant SKOV-3 and OVCAR-8 cells ovarian cancer cells.

### Materials and Methods

Viability of SKOV-3 cells before and after various Septin-2 targeting antisense PTOs at different concentrations treatment was determined by the 96®Aqueous-One-Solution Assay (Promega, Madison, WI). Briefly, SKOV-3 or OVCAR-8 cells (5000/well) were plated into 96 well flat bottom plates (Corning, Inc., Corning, NY) overnight. The cells were treatment with various dilutions of Septin-2 targeting antisense PTOs (PTOs 1-4) in sterile PBS or vehicle (PBS) as indicated. Following incubation at 37°C in a cell culture incubator for 20 h MTS reagent was added at a 1:10 dilution to the medium. The samples were incubated for an additional 4 h before absorbance was measured at 490 nm in an ELISA plate reader (Thermo Labsystems, Waltham, MA). Experiments were performed in triplicates; data are expressed as the mean of the triplicate determinations of a representative experiment in % of absorbance by samples with untreated cells (= 100%).

### Sequence of the oligos:

**PTO-1:** 5'-T*T*C* G*C*C* T*C*T* C*C*T* C*C*T* G*T*C* C*T-3'
**PTO-2:** 5'-T*C*C* A*C*T* T*T*C* C*T*G* C*C*G* C*C*T* C* T-3'
**PTO-3:** 5'-C*C*T* C*T*C* C*T*C* C*T*G* T*C*C* T*C*C* A*A-3'
**PTO-4:** 5'- T*C*T* C*C*A* C*T*T* C*C*A* C*A*A* C*A*C* C*C-3'
**PTO-5:** 5'- T*C*C* T*C*C* C*T*C* C*T*C* G*C*A* T*A*G* C*T-3'
**PTO-6:** 5'- T*C*T* C*C*A* T*C*T* C*C*C* T*A*C* A*C*A* A*C*C-3'
**PTO-7:** 5'- T*C*G* T*C*T* C*A*C* C*T*C* T*G*C* T*G*C* C*A-3'

Ovarian cancer cell lines were created that stably expressed septin-2 shRNAs. SKOV-3 cells were transfected with Septin-2 shRNA (Santa Cruz biotechnology proprietary method) or a null oligo. The cells were selected under antibiotic pressure. Single cells were selected from among the surviving cells and clones (KO-9 and KO-11) that expressed significantly lower Septin-2 were selected and grown into colonies and characterized. The proliferation rate of the KO-9 and KO-11 clones compared to wild type and null vector inserted SKOV-3 cells was estimated by counting the cell numbers at defined hours after seeding. Briefly, 100000 cells of SKOV-3 wt, nv, KO-9 and KO-11 were seeded in a 100cm3 petri-dish in complete DMEM media (7mL each) in six replicates. Cells in petri dishes were counted at 72^{nd} hour in the first three replicates and at 96th hour in the remaining three replicated.

### Results

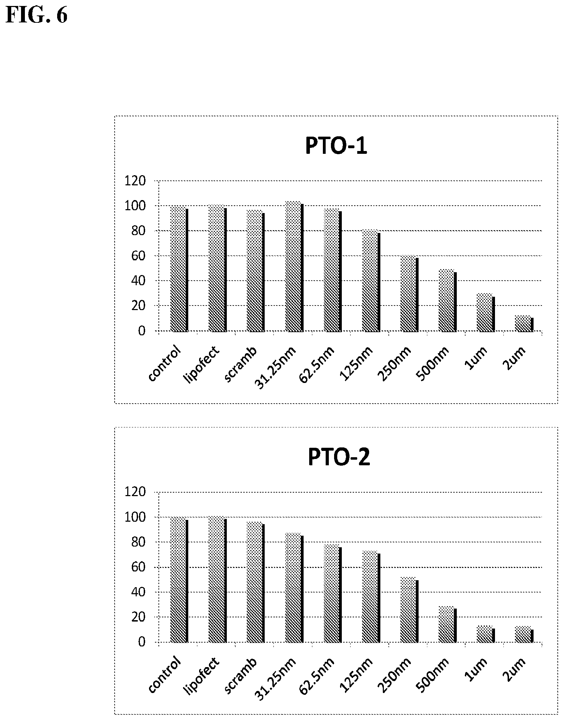

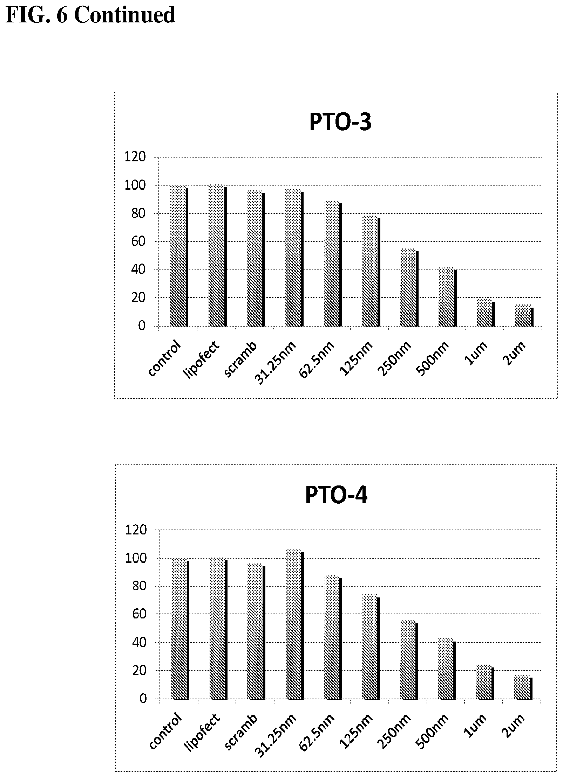

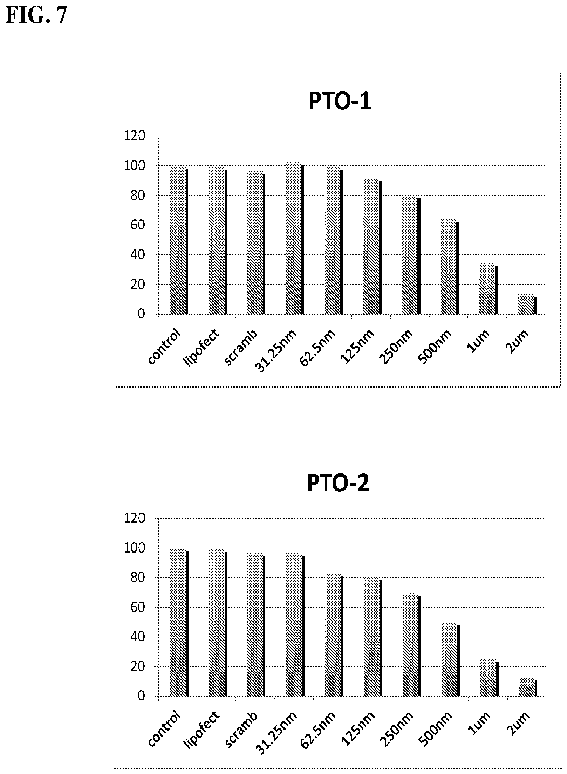

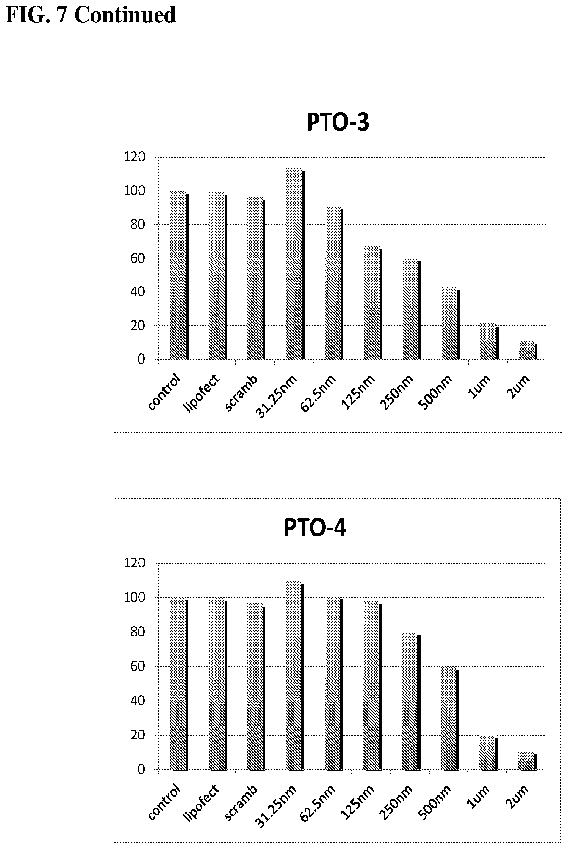

As shown in Figs. 6 and 7, treatment with diversely modified antisense PTOS target Septin-2 expression dose-dependently inhibited the viability of both SKOV-3 and ovcar-resulted in cytotoxic effects on platinum resistant ovarian cancer cell-lines during 24 hours of treatment.

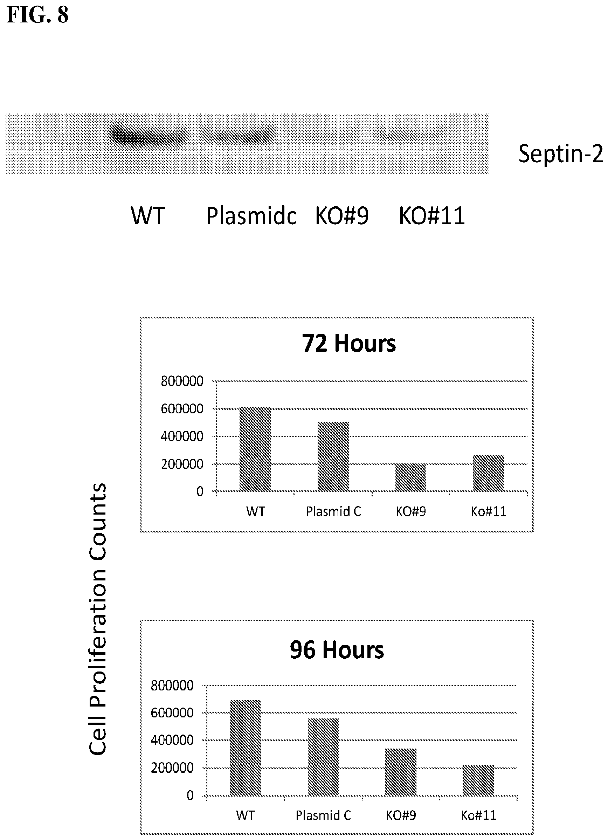

SKOV-3 cells were then engineered to stably express a shRNA directed to septin-2. As shown in **FIG. 8**, stable septin-2 inhibition reduced proliferation of SKOV-3 cells after both 72 and 96 hours in culture. As such, this Example demonstrates that knock down of septin-2 expression can decrease ovarian cancer cellular proliferation.

### Example 5

This Example shows that increased HE4 levels are correlated with increased septin-2 expression in ovarian cancer cells.

### Materials and Methods:

SKOV-3 wt, NV, and HE4 overexpressing clones C1 and C1, or OVCAR-8 cells wt, NV, and HE4 overexpressing clone-5 were seeded and allowed to adhere overnight in Lab-Tek 8-well chamber slides. Cells were fixed with 10% neutral buffered formalin, permeabilized, and washed with PBS-Tween 20 0.05% (Sigma-Aldrich) three times. 5% horse serum (Sigma-Aldrich) in PBS-Tween was used to block slides and dilute antibodies. Cells were incubated with Septin-2 primary antibody overnight at 4°C, washed with PBS-Tween (3×5 minutes), and incubated with Alexa flour secondary antibody) for 1 hour at room temperature in the dark. Slides were washed with PBS-Tween (3×5 minutes) and cover-slipped with DAPI-containing mounting medium (Vector Labs, Burlingame, CA, USA). Confocal images were obtained and processed. Western blots were performed using a septin-2 primary antibody according to standard protocols.

For immunoprecipitation studies, two million SKOV3 WT and OVCAR-8 cells were seeded onto 100 mm dishes (Corning) and cultured to 80% confluency in complete DMEM media. Cells were rinsed in DPBS (Gibco), lysed, and quantified using Bradford assay. Lysates were adjusted to 1 µg/µl total protein concentration, with 500 µl lysate used for each IP reaction. Identical samples were prepared for pull-down antibody (Septin-2 or HE4) and isotype-matched IgG control (#3900, Cell Signaling), with antibody concentration identical at 40 ng/µl. Further processing and PAGE analysis of samples was conducted to resolve the bands. The resulting membranes were probed for HE4 or Septin-2, p21 or P53, B-tubulin and other proteins of interest.

### Results:

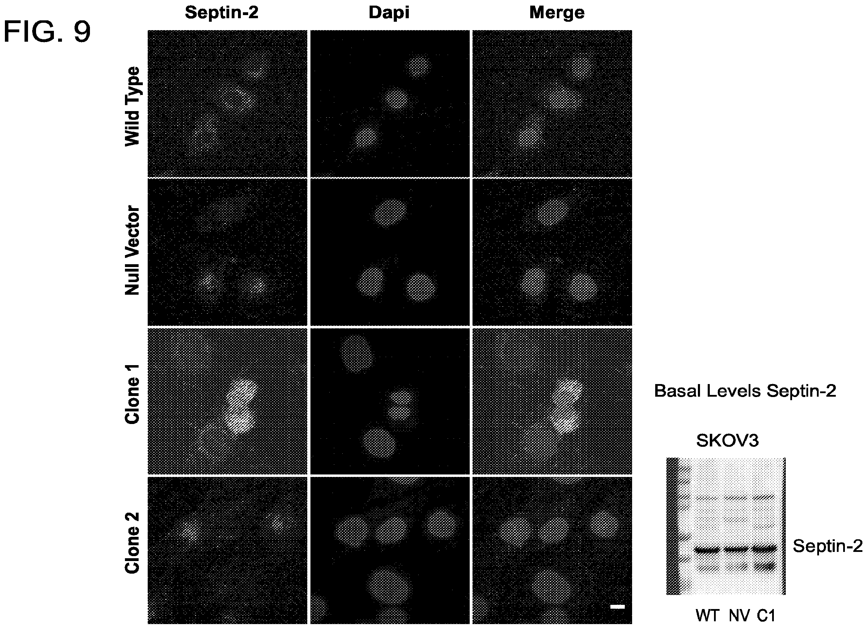

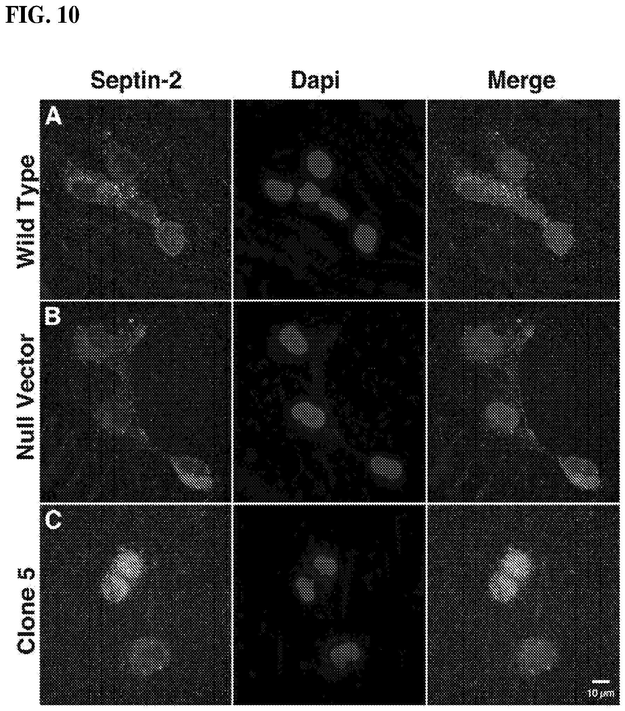

As shown in **FIGS. 9** **and** **10****,** overexpression of HE4 leads to increased septin-2 expression. Stable HE4-expressing SKOV-3 cells clones (clone-1 and clone-2) along with wild-type and null vector were fixed and stained with septin-2 antibody and a corresponding Alexa Fluor antibody. The confocal images were acquired. Dividing cells express elevated septin-2 among the cells that stably overexpress HE4.

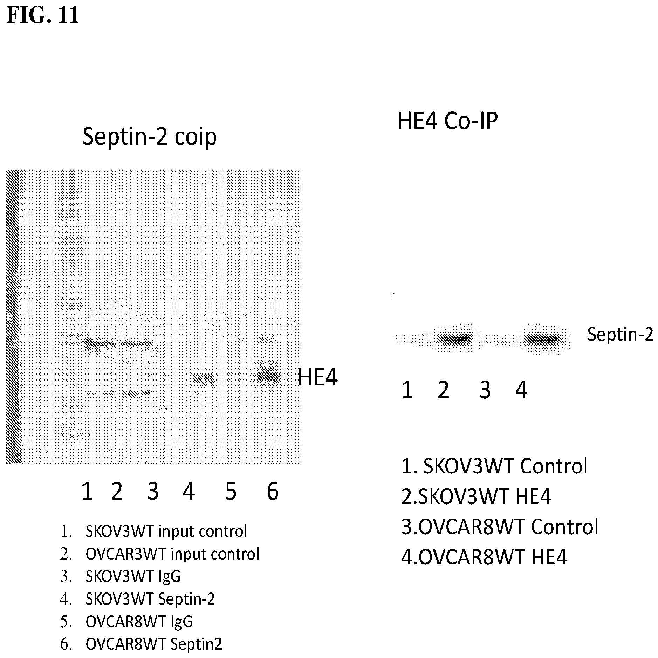

Given that increased expression of HE4 correlates with a similar increase in expression of septin-2, the potential interaction between these proteins in ovarian cancer cells was investigated. As shown in **FIG. 11****,** immunoprecipitation of septin-2 captured HE4 in both SKOV3 WT and OVCAR-8 cell lysate. Similar results were obtained when HE4 was used as the capture antibody to pull down septin-2 **(****FIG. 11**).

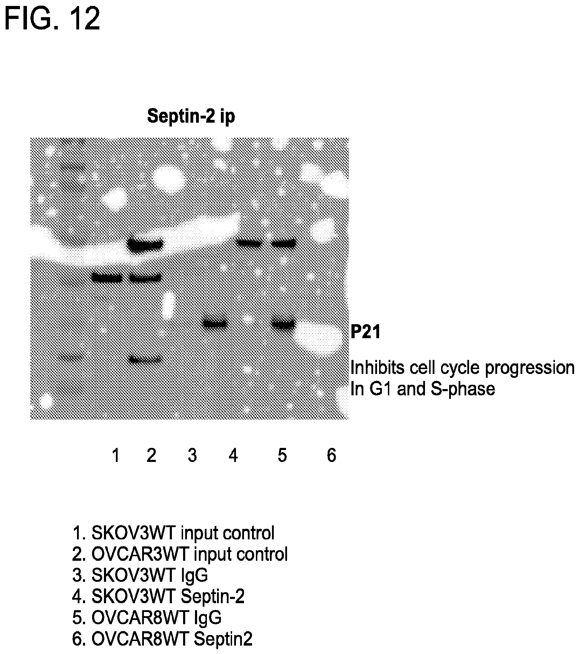

A further immunoprecipitation experiment showed that septin-2 is associated with cyclin dependent kinase inhibitor (p21) in SKOV-3 and OVCAR-8 cells. As shown in **FIG. 12****,** p21 was found in SKOV3 WT and OVCAR-8 cells lysates immunoprecipitated with a septin-2 pull-down antibody. However additional immunoprecipitation experiments using septin-2 as a pull-down antibody failed to capture either p53 or β-tubulin (**FIG**. **13**).

Overall, these results show that increased HE4 expression is associated with increased expression of septin-2 in ovarian cancer cells and that septin-2 co-immunoprecipitates with both HE4 as well as the cyclin dependent kinase inhibitor (p21).

### Example 6

This Example shows that septin-2 is selectively overexpressed in clear cell EOC.

### Materials and Methods

Fluorescent immunohistochemistry for detection of septin-2 in ovarian tissue sections as well as quantification of signal intensity is performed as described above. DNA was stained with DAPI.

### Results:

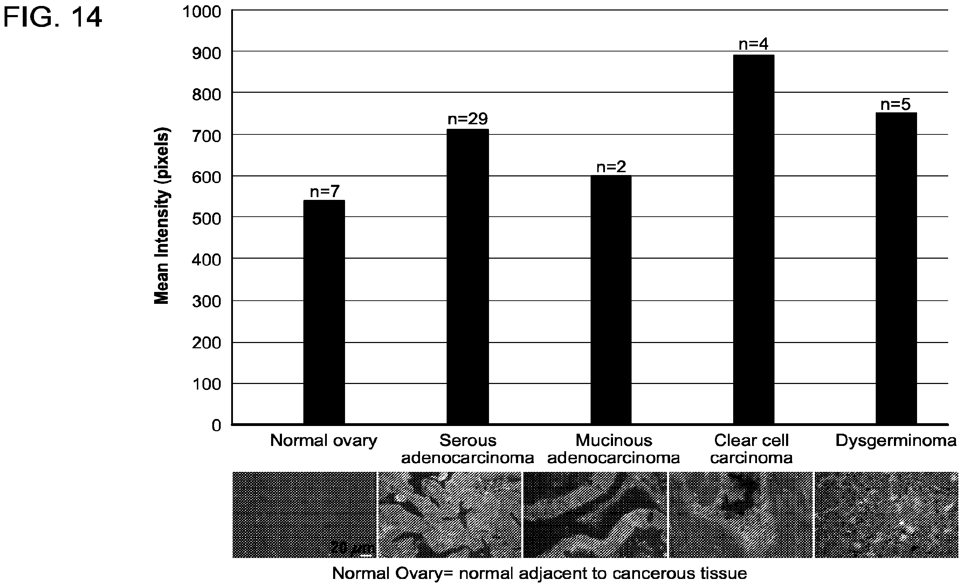

An ovarian tumor tissue array was fluorescently stained with a septin-2 specific antibody. Tissue from normal ovary, serous adenocarcinoma, mucinous adenocarcinoma, clear cell carcinoma, and dysgerminoma were examined. As shown **in** **FIG. 14****,** tumors characterized as clear cell carcinoma exhibited the highest intensity staining.

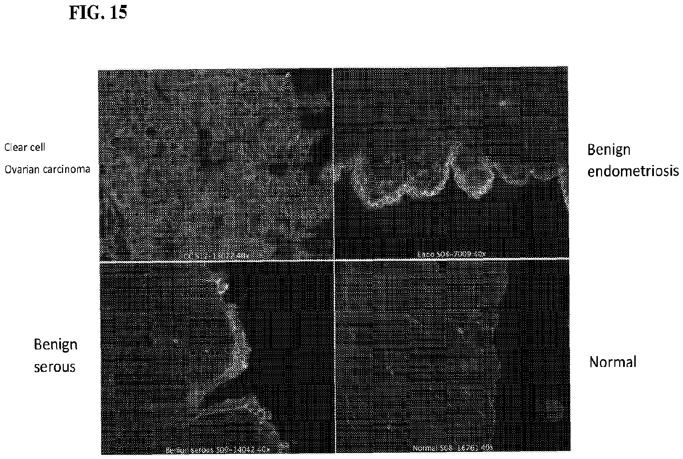

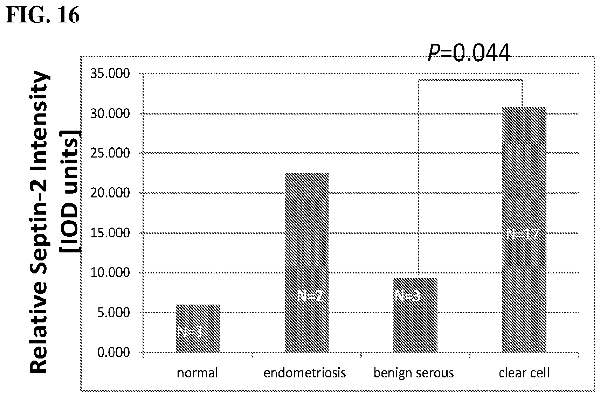

Based on the results of the ovarian tissue array, the expression of septin-2 expression was examined in a number of other types of ovarian tumors characterized by increased growth. As shown in **FIGS. 15** and **16****,** Septin-2 is overexpressed in clear cell ovarian carcinoma tissue compared to its expression in benign serious, benign endometriotic, and normal ovarian tissues.

### SEQUENCES

Septin-2 protein sequence (SEQ ID NO:1)

MSKQQPTQFI NPETPGYVGF ANLPNQVHRK SVKKGFEFTL MVVGESGLGK

STLINSLFLT DLYPERVIPG AAEKIERTVQ IEASTVEIEE RGVKLRLTVV

DTPGYGDAIN CRDCFKTIIS YIDEQFERYL HDESGLNRRH IIDNRVHCCF

YFISPFGHGL KPLDVAFMKA IHNKVNIVPV IAKADTLTLK ERERLKKRIL

DEIEEHNIKI YHLPDAESDE DEDFKEQTRL LKASIPFSVV GSNQLIEAKG

KKVRGRLYPW GVVEVENPEH NDFLKLRTML ITHMQDLQEV TQDLHYENFR

SERLKRGGRK VENEDMNKDQ ILLEKEAELR RMQEMIARMQ AQMQMQMQGG

DGDGGALGHH V

Septin-2 nucleic acid sequence (SEQ ID NO:2)

1 aggaaggcca cgtccccggg gagggacgcc cgactcgatg gtctgcgcag ggcccgtcgg
61 ccccacgtga cgtgcgcgca gccaatccca gagaggcccc ccgatcatcg agacggcggc
121 ggtggtgggc tagacgagtt tcgcgcggcc gctcgccgtc ccccgcccag tcgtactcgg
181 cgccccagct cggtgctgcc gccatcttct tggaggacag gaggagaggc gaaggctccc
241 cctccccgtg atcgctccgc actcccgcca ccacctgccc tcccgcgacc gcctctctcc
301 tcctcagtgg gcacttgtct ccttctaaca aacggccttc cccccactcc agttacccac
361 cgcaaggcga agattctcat tacctgttcc actcttataa gcataagaaa accgagctca
421 taagcataca gaaactgctg taaaagaaga agttgtgggt attttttttt ttttttttgt
481 ctggaggaat ggggacacca aaactcattt ggcagcagag gtgagacgaa gcttcacaaa
541 agatgtctaa gcaacagcca actcagttta taaatccaga aacacctggc tatgttggat
601 ttgcaaacct ccccaatcaa gttcaccgaa aatcagtgaa aaaaggtttt gagttcacac
661 tgatggtggt cggtgaatca ggtctaggaa aatcgactct cataaacagc ctattcctaa
721 ctgatctgta cccagaaaga gtcatacctg gagcagcaga aaaaattgaa agaactgtcc
781 agattgaggc ttcaactgtt gaaattgaag agcgaggggt caagctacgc ctgacagtgg
841 tagatacccc tggctatggt gacgctatca actgcagaga ttgttttaag acaattatct
901 cctatattga tgagcaattt gagaggtacc tgcatgacga gagcggcttg aacaggcggc
961 acatcattga taatagggtg cattgttgct tttactttat ttcacctttt ggacatggac
1021 ttaagccctt agatgtggcg tttatgaagg caatacacaa caaggtgaat attgtgcctg
1081 tcattgcaaa agctgacact ctcaccctga aggaacggga gcggctgaag aaaaggattc
1141 tggatgaaat tgaagaacat aacatcaaaa tctatcactt acctgatgca gaatcagatg
1201 aagatgaaga ttttaaagag cagactagac ttctcaaggc tagcatccca ttctctgtgg
1261 ttggatccaa tcagttgatt gaagccaaag gaaagaaggt cagaggccgc ctctacccct
1321 ggggtgttgt ggaagtggag aacccagagc acaatgactt tctgaagctg agaaccatgc
1381 tcatcaccca catgcaggat ctccaggagg tgacccagga ccttcattat gaaaacttcc
1441 gttctgagag actcaagaga ggcggcagga aagtggagaa tgaggacatg aataaagacc
1501 agatcttgct ggaaaaagaa gctgagctcc gccgcatgca agagatgatt gcaaggatgc
1561 aggcgcagat gcagatgcag atgcagggcg gggatggcga tggcggggct ctcgggcacc
1621 acgtgtaagg tgatgtgcac atatcaagaa gtcagagaaa acactttcct ggataaaaaa
1681 gaaaacattc cagatgcatg atccagctgt gtgttttcaa tccttgggag ggtgccatcc
1741 acattttaac agtacctgtg cctgagaatt taatttttaa aagactttga tgtgtttttg
1801 tatgaagtac ttttaacgta tgtatttcat tgctgtgtca cactctgtgt tttgtgaggt
1861 gaatgtcttc cttttctttc tccctaacca ctaatgttag aattgatttc caagaatcgg
1921 catgtatact taatactgaa tttctttgat ttaactgact taacaactga ctaaccattg
1981 atgagcactc ctgattttta tctagaacat tcagatttac cataatgttc cttagtggta
2041 gaggtgtgtg cctagtgatg tagaaagata cactgacttg gtgcaaggcc atctgcttac

2101 cacatcacac cacttggaga tctttgcttc cttgctttta tgtttgtaca caacacctaa
2161 aaccagtttt gctgctataa ttctatactg ttgattcgtc tgcgatttta tctgttaacc
2221 aaataaaaca taatagaatt tcctaatgag atatatcttt atacttaaac agctttttta
2281 gaggtgagtt ttaaagaagt ctcttaattc tgatgctagg ttgtttttaa aaccactatg
2341 caaagaactc accacaagcc accttttgta gtgttctcca ctaatactgg ttatcctgtg
2401 ctacagagaa aatcaaagca gtcataagct ccagttttcg tattgcaaat aagactctta
2461 cctacaaaat gagattcagt gaactaattt ggtttttact caaccaaatt aaaaattttt
2521 ttaaggaaaa ttagcagttg gtctattcag aatcaaacct ttttatattt tatactgcac
2581 tttagtgtat tttctgtcac tgtaggtata gaagatctgc ctcccctgtg gaaattgggg
2641 tctgttggtg ggcgtgcccc tgaagcctgg cttgggttga aaagtgttcc cgccctaagg
2701 ccttggtgcc ctgaacctct gatgcctacc gggttctcct gatttgagtt tcctttaaat
2761 actccctttt tgagtaattt tctgatggga ggaaagtagc agtcatcatc tttttgtgtg
2821 caggctgtct catttatttt tagccattgt cgtttcattc attttgtgta atataaaccg
2881 tgtgtcatgt caaagtgaaa gacatttcaa atctgtagca taggctagtg ggcaggtccg
2941 cacagtcgaa gccacacctg gtctgttttc tgtgcactgt agccttagtg tcacctttct
3001 tcttgtgtct ccttatggta cactccagcg gttgcctttt ttatcatttc tactgaagtt
3061 gggaaattca accccagaaa ttgacagatg aaaggagaca atggttgtgt agggagatgg
3121 agaaaatgct taatctgagg atgagacagg gttttttcat ttttgtgggg gctagaaaaa
3181 acataaaatg aggcagttaa ataataatag ttaatgaagg tgtgctacag aaaataatct
3241 ggtgttcttg ctaactttgc ccttcactgt tgcttaattg tgaacagcca aaagctatat
3301 gttatggctt attgtgtgaa ggtaactaag aagtggtgtt ccatgacttc agagtacatc
3361 catgcggagt ccattatttg agtttgacat ttaataactt tgctggaaaa tctgtaaaaa
3421 agaaaaacaa gtttgctagt gactaagccc cgcatatgtg agtgaaagta cttcaggcac
3481 gctgcctcct ggtaacagct atgcagggag ggaggaccca cactgctaca cttctgatcc
3541 cctttggttt tactacccaa atctaaatag atacttttga taatagataa ctgctctttt
3601 actaagacat agtctctacc tatagaaatg tattttgaaa acacttattt tacacagcaa
3661 ttttgtatcc atttaaacta accttttatc aataaagcac tattgtttag atattaaaaa
3721 aaaaaa

## Claims

1. A method for diagnostically evaluating a subject for Müllerian cancer, said method comprising: measuring the expression of a septin family gene or protein in a sample from the subject, wherein the subject is diagnosed with Müllerian cancer if the expression of the septin family gene or protein is higher in the sample than in one or more control samples acquired from one or more subjects without Müllerian cancer,
wherein the septin family gene or protein septin-2 as set forth in SEQ ID NO: 2 and 1, respectively.

2. The method of claim 1,
i) wherein the method further comprises measuring the expression of a gene or protein of one or more biomarkers selected from the group consisting of CA125, p21, HE4, transthyretin, LPA, YKL-40 and inhibin, wherein the subject is diagnosed with Müllerian cancer if a) the expression of the septin family gene or protein and b) the expression of said gene or protein of said one or more biomarkers is higher in the sample than in said one or more control samples acquired from one or more subjects without Müllerian cancer; and/or
ii) wherein said subject is pre- or post-menopausal; and/or
iii-1) wherein said subject has been diagnosed as having a Müllerian cancer and said method is used to determine if said Müllerian cancer has recurred or advanced or
iii-2) wherein said subject has not been previously diagnosed as having a Müllerian cancer and said method is used to evaluate whether a Müllerian cancer is present; and/or
iv) wherein the expression of said septin family gene or protein in said sample is at least 1-500 times higher compared to one or more control samples; and/or
v) wherein the expression of said septin family gene or protein in said sample is at least 1.3-8 fold higher compared to one or more control samples; and/or
vi) wherein the expression of said septin family gene or protein in said sample is at least 6 fold higher compared to one or more control samples.

3. The method of any one of claims 1-2, wherein the expression of said septin-2 gene or protein in said sample is at least 3-6 folds higher compared to one or more control samples.

4. The method of any one of claims 2-3,
i) wherein the expression of said septin-2 gene or protein said sample is at least 3-6 folds higher compared to one or more control samples and the expression of said HE4 gene or protein in said sample is at least 4-8 folds higher compared to one or more control samples; or
ii) wherein the expression said septin-2 gene or protein in said sample is at least 3-6 folds higher compared to one or more control samples and the expression of one or more of CA125, p21, transthyretin, LPA, YKL-40 and/or inhibin gene or protein in said sample is at least 4-8 folds higher compared to one or more control samples.

5. The method of any one of claims 3-4,
i) wherein the expression of said septin-2 gene and/or said biomarker protein is measured by immunohistochemistry, ELISA, RIA, Western or immunoblot, or another quantitative antibody-based method, particularly wherein said antibody is an antibody generated against the polypeptide encoded by SEQ ID NO:1 especially wherein the antibody is a monoclonal antibody or a functional fragment thereof; or
ii) wherein the expression of said septin-2 gene and/or said biomarker protein expression is measured by mass spectrometry or chromatography; or
iii) wherein the expression of said septin-2 gene and/or said biomarker gene expression is measured by qRT-PCR, RT- PCR or another PCR-based method, Northern Blot, or SAGE.

6. A method for stratifying a subject with a pelvic mass for the risk of having or developing a Müllerian cancer, the method comprising measuring the expression of a septin family gene or protein in a sample from the subject, wherein the subject is at increased risk of having or developing a Müllerian cancer if the expression of the septin family gene or protein is higher in the sample than in one or more control samples acquired from one or more subjects without a Müllerian cancer,
wherein the septin family gene or protein is septin-2 as set forth in SEQ ID NO: 2 and 1, respectively
particularly
i) wherein the method further comprises measuring the expression of one or more biomarkers selected from the group consisting CA125, p21, HE4, transthyretin, B2-Microglobulin, apolipoprotein A-1, prealbumin, transferrin, LPA, YKL-40, follicle stimulating hormone (FSH), anti-Müllerian Hormone (AMH)and inhibin, wherein the subject is at increased risk for having or developing Müllerian cancer if a) the expression of the septin family gene or protein and b) the expression of said one or more biomarkers is higher in the sample than in said one or more control samples acquired from one or more subjects without a Müllerian cancer; and/or
ii-1) wherein a) increased expression of the septin family gene or protein or b) increased expression of the septin family gene or protein expression and increased expression of said one or more biomarkers signifies that the subject is at increased risk for one or more of malignant cancer or early stage malignant Müllerian cancer, or ii-2) wherein a) equivalent expression of the septin family gene or protein or b) equivalent expression of the septin family gene or protein and equivalent expression of said one or more biomarkers signifies that the pelvic mass is benign or a low malignant potential tumor (LMP).

7. A method for classifying a pelvic mass in a subject at risk of having or developing a Müllerian cancer, the method comprising measuring the expression of a septin family gene or protein in a sample from the subject, wherein the pelvic mass is classified as being malignant if the expression of the septin family gene or protein is higher in the sample than in one or more control samples acquired from subjects without a Müllerian cancer,
wherein the septin family gene or protein or fragment thereof is septin-2, as set forth in SEQ ID NO: 2 and 1, respectively
particularly
A1) wherein the method further comprises:
(i) measuring the quantity of CA125 and/or HE4 in the sample;
(ii) comparing the quantity of the septin family gene or protein and the quantity of CA125 and/or HE4 as measured in (i) with a reference value of the quantity of the septin family gene or protein and the quantity of CA125 and/or HE4, wherein the reference value represents a known classification of a pelvic mass, Müllerian tumor, or Müllerian cancer;
(iii) finding a deviation or no deviation of the septin family gene or protein and the quantity of CA125 and/or HE4 as measured in (i) from said reference value in (ii); and
(iv) classifying the pelvic mass in said subject as being benign or malignant based on said finding of deviation or no deviation; or
A2) wherein the method further comprises
(i) measuring the quantity of (a) a septin family protein and (b) HE4 and/or CA125 in a sample from the subject;
(ii) entering the quantities measured in (i) into an equation wherein each quantity is given a weight; and
(iii) analyzing whether the numerical value obtained in (ii) falls within a range of benign to malignant Müllerian cancer, wherein said ranges of benign, early malignant, borderline, or malignant Müllerian cancer have been established by using the same equation on samples from subjects for which respectively benign or malignant Müllerian cancer has been previously diagnosed or classified, especially wherein said malignant Müllerian cancer is early malignant, or borderline; and/or
B) wherein the method further comprises: measuring the quantity of one or more other biomarkers in the sample from said subject, wherein said other biomarker is selected from the group consisting of mesothelin, CA72-4, osteopontin, CA125, p21, HE4, transthyretin, B2-Microglobulin, apolipoprotein A-1, prealbumin, transferrin, LPA, YKL-40, follicle stimulating hormone (FSH), anti-Müllerian Hormone (AMH) and/or inhibin.

8. The method of any one of claims 6-7, further comprising morphologically analyzing the pelvic mass by a method selected from the group consisting of one or more of ultrasound (US), magnetic resonance imaging (MRI), computed tomography (CT),and positron emission tomography-computed tomography (PET-CT).

9. The method of any one of claims 1-8,
i) wherein said method further comprises assessing risk factors in the subject selected from the group consisting of genetic predisposition due to mutations in the BRCA gene family, familial predisposition, age, diet, obesity, reproductive history, menopausal status, gynecological surgery, hormonal replacement therapy, smoking and alcohol use, particularly wherein gynecological surgery comprises tubal ligation or hysterectomy; and/or
ii) wherein said method is used at regular time points to follow the expression of a) the septin family gene or protein and/or b) the septin family gene or protein and said biomarker(s) in combination with the risk factors during the life of the subject.

10. A method for detecting a change in the prognosis for a subject diagnosed with a Müllerian cancer comprising measuring the expression of a septin-2 gene or protein as set forth in SEQ ID NO:2 and 1, respectively, in a sample from the subject during or after treatment for the Müllerian cancer, wherein a change in the expression level of septin-2 in comparison with a reference value for expression of a septin-2 gene, protein, or fragment thereof in one or more subjects with benign tumors indicates a change in the prognosis for the subj ect.

11. The method of claim 10, wherein the subject is pre- or post-menopausal.

12. The method of any one of claims 1-11,
i) wherein said sample is blood, serum, plasma, tissue, or urine; and/or
ii) wherein said Müllerian cancer is selected from the group consisting of ovarian, fallopian tube, primary peritoneal, endometrial and uterine cancers, particularly wherein said ovarian cancer or ovarian tumor is selected from the group consisting of one or more of an epithelial carcinomas, malignant sex cord stromal tumor, malignant germ cell tumors, metastatic carcinoma infiltrated in the pelvis or in the ovaries, cystadenoma, fibroma, thecoma, cystadenofibroma, mature teratoma, endometriosis, follicular cyst, abscess, struma ovarii, Leydig cell tumor, parasalpingeal cyst, hydrosalpinx, corpus luteum cyst, clear cell ovarian carcinoma, hemorrhagic cyst, tissue with calcifications NOS, necrotic tumor NOS or combinations thereof.

13. The method of any one of claims 6-12,
i) wherein septin family protein expression is measured, particularly a) wherein the septin family protein expression is measured by immunohistochemistry, ELISA, RIA, Western or immunoblot, or another antibody-based method, or b) wherein the septin family protein expression is measured by mass spectrometry or chromatography; or
ii) wherein septin family gene expression is measured, particularly a) wherein septin family gene expression is measured by qRT-PCR, RT- PCR or another PCR-based method, Northern Blot or SAGE, or b) wherein DNA methylated forms of septin family genes, isoforms of septin family genes, circulating septin family DNA, or microRNA or fragments thereof are measured.

14. An agent for use in a method for treating a proliferative disease in a subject, the method comprising inhibiting the expression or activity of a septin family member gene, protein, or fragment thereof;
wherein the septin family gene or protein is septin-2, as set forth in SEQ ID NO: 2 and 1, respectively, wherein the proliferative disease is Müllerian cancer,
i) wherein said inhibition results in an antitumor, anticancer, anti-proliferative, anti-angiogenic, or anti-lipogenic effect; and/or
ii-1) wherein septin family member gene expression is inhibited by administration of an effective amount of one or more agents that specifically bind the septin-2 gene,
wherein said agent is selected from the group consisting of an antisense oligonucleotide, a siRNA, and a phosphorothio oligonucleotide (PTOs), or ii-2) wherein septin family member protein or fragment thereof expression is inhibited by administration of an effective amount of an antibody or fragment thereof, wherein said antibody or fragment thereof specifically binds the septin-2 protein.

15. A kit comprising
(i) means configured to measure (a) the quantity of the septin-2 protein as set forth in SEQ ID NO: 1 and (b) the quantity of CA125, HE4 and/or one or more other biomarkers in a sample from a subject, wherein said other biomarker is selected from the group consisting of mesothelin, CA72-4, osteopontin, CA125, p21, HE4, transthyretin, B2-Microglobulin, apolipoprotein A-1, prealbumin, transferrin, LPA, YKL-40, follicle stimulating hormone (FSH), anti-Müllerian Hormone (AMH) and/or inhibin; and
(ii) a reference value of (a) the quantity of septin-2 and (b) the quantity of CA125, HE4 and/or one or more of the other above mentioned biomarkers, wherein said reference value represents a known classification of a Müllerian tumor.

## Patentansprüche

1. Verfahren zur diagnostischen Beurteilung eines Subjektes auf Müller'schen Krebs, wobei das Verfahren umfasst: Messen der Expression von einem Septin-Familie-Gen oder -Protein in einer Probe von dem Subjekt, wobei das Subjekt mit Müller'schem Krebs diagnostiziert wird, wenn die Expression von dem Septin-Familie-Gen oder (-)Protein in der Probe höher ist als in ein oder mehreren Kontrollproben, erworben von ein oder mehreren Subjekten ohne Müller'schen Krebs,
wobei das Septin-Familie-Gen oder -Protein Septin-2, wie in SEQ ID NO: 2 bzw. 1 dargelegt, ist.

2. Verfahren nach Anspruch 1,
i) wobei das Verfahren weiterhin Messen der Expression von einem Gen oder Protein von ein oder mehreren Biomarkern, ausgewählt aus der Gruppe, bestehend aus CA125, p21, HE4, Transthyretin, LPA, YKL-40 und Inhibin, umfasst, wobei das Subjekt mit Müller'schem Krebs diagnostiziert wird, wenn a) die Expression von dem Septin-Familie-Gen oder -Protein und b) die Expression von dem Gen oder Protein von den ein oder mehreren Biomarkern in der Probe höher ist als in den ein oder mehreren Kontrollproben, erworben von ein oder mehreren Subjekten ohne Müller'schen Krebs; und/oder
ii) wobei das Subjekt prä- oder postmenopausal ist; und/oder
iii-1) wobei das Subjekt diagnostiziert worden ist, Müller'schen Krebs zu haben, und das Verfahren verwendet wird, um zu beurteilen, ob der Müller'sche Krebs wiederaufgetreten ist oder fortgeschritten ist oder
iii-2) wobei das Subjekt zuvor nicht diagnostiziert worden ist, Müller'schen Krebs zu haben, und das Verfahren verwendet wird, um zu beurteilen, ob ein Subjekt mit Müller'schem Krebs vorhanden ist; und/oder
iv) wobei die Expression von dem Septin-Familie-Gen oder (-)Protein in der Probe verglichen mit ein oder mehreren Kontrollproben mindestens 1-500 mal höher ist; und/oder
v) wobei die Expression von dem Septin-Familie-Gen oder (-)Proteins in der Probe verglichen mit ein oder mehreren Kontrollproben mindestens 1,3-8 mal höher ist; und/oder
vi) wobei die Expression von dem Septin-Familie-Gen oder (-)Protein in der Probe verglichen mit ein oder mehreren Kontrollproben mindestens 6 mal höher ist.

3. Verfahren nach einem beliebigen der Ansprüche 1-2, wobei die Expression von dem Septin-2-Gen oder (-)Proteins in der Probe verglichen mit ein oder mehreren Kontrollproben mindestens 3-6 mal höher ist.

4. Verfahren nach einem beliebigen der Ansprüche 2-3,
i) wobei die Expression von dem Septin-2-Gen oder (-)Protein in der Probe verglichen mit ein oder mehreren Kontrollproben mindestens 3-6 mal höher ist, und die Expression von dem HE4-Gen oder -Protein in der Probe verglichen mit ein oder mehreren Kontrollproben mindestens 4-8 mal höher ist; oder
ii) wobei die Expression von dem Septin-2-Gen oder (-)Protein in der Probe verglichen mit ein oder mehreren Kontrollproben mindestens 3-6 mal höher ist und die Expression von ein oder mehreren von dem CA125-, p21-, Transthyretin-, LPA-, YKL-40- und/oder Inhibin-Gen oder -Protein in der Probe verglichen mit ein oder mehreren Kontrollproben mindestens 4-8 mal höher ist.

5. Verfahren nach einem beliebigen der Ansprüche 3-4,
i) wobei die Expression von dem Septin-2-Gen und/oder Biomarker-Protein durch Immunohistochemie, ELISA, RIA, Western- oder Immuno-Blotting oder ein anderes quantitatives auf Antikörper(n) basiertes Verfahren gemessen wird, insbesondere wobei der Antikörper ein Antikörper, erzeugt gegen das von SEQ ID NO: 1 kodierte Polypeptid ist, insbesondere wobei der Antikörper ein monoklonaler Antikörper oder ein funktionales Fragment davon ist; oder
ii) wobei die Expression von dem Septin-2-Gen und/oder die Biomarker-Protein-Expression durch Massenspektrometrie oder (-)Chromatographie gemessen wird; oder
iii) wobei die Expression von dem Septin-2-Gen und/oder die Biomarker-GenExpression durch qRT-PCR, RT- PCR oder ein anderes PCR-basiertes Verfahren, Northern Blotting oder SAGE gemessen wird.

6. Verfahren zum Stratifizieren eines Subjekts mit einer Beckenmasse ("pelvic mass") auf das Risiko, einen Müller'schen Krebs zu haben oder zu entwickeln, wobei das Verfahren Messen der Expression von einem Septin-Familie-Gen oder (-)Protein in einer Probe von dem Subjekt umfasst, wobei das Subjekt einem erhöhten Risiko ausgesetzt ist, einen Müller'schen Krebs zu haben oder zu entwickeln, wenn die Expression von dem Septin-Familie-Gen oder (-)Protein in der Probe höher ist als in ein oder mehreren Kontrollproben, erworben von ein oder mehreren Subjekten ohne einen Müller'schen Krebs ,
wobei das Septin-Familie-Gen oder -Protein Septin-2, wie in SEQ ID NO: 2 bzw. 1 dargelegt, ist,
insbesondere
i) wobei das Verfahren weiterhin Messen der Expression von ein oder mehreren Biomarkern, ausgewählt aus der Gruppe, bestehend aus CA125, p21, HE4, Transthyretin, B2-Mikroglobulin, Apolipoprotein A-1, Prä-Albumin, Transferrin, LPA, YKL-40, Follikel-stimulierendem Hormon (FSH), anti-Müller'schem Hormone (AMH) und Inhibin, umfasst, wobei das Subjekt einem erhöhten Risiko ausgesetzt ist, einen Müller'schen Krebs zu haben oder zu entwickeln, wenn a) die Expression von dem Septin-Familie-Gen oder (-)Protein und b) die Expression von dem ein oder mehreren Biomarker in der Probe höher ist als in den ein oder mehreren Kontrollproben, erworben von ein oder mehreren Subjekten ohne einen Müller'schen Krebs; und/oder
ii-1) wobei a) eine erhöhte Expression von dem Septin-Familie-Gen oder (-)Protein oder b) erhöhte Expression von dem Septin-Familie-Gen oder (-)Protein-Expression und erhöhte Expression von den ein oder mehreren Biomarkern bedeutet, dass das Subjekt einem erhöhten Risiko für ein oder mehrere bösartige Krebsarten oder bösartigen Müller'schen Krebs im Frühstadium ausgesetzt ist, oder ii-2) wobei a) äquivalente Expression von dem Septin-Familie-Gen oder (-)Protein oder b) äquivalente Expression von dem Septin-Familie-Gen oder (-)Protein und äquivalente Expression von den ein oder mehreren Biomarkern bedeuten, dass die Beckenmasse gutartig oder ein Tumor mit geringem bösartigem Potenzial (LMP) ist.

7. Verfahren zum Klassifizieren einer Beckenmasse in einem Subjekt mit Risiko, einen Müller'schen Krebs zu haben oder zu entwickeln, wobei das Verfahren Messen der Expression von einem Septin-Familie-Gen oder (-)Protein in einer Probe von dem Subjekt umfasst, wobei die Beckenmasse klassifiziert wird, bösartig zu sein, wenn die Expression von dem Septin-Familie-Gen oder (-)Protein höher ist in der Probe als in ein oder mehreren Kontrollproben, erworben von ein oder mehreren Subjekten ohne einen Müller'schen Krebs,
wobei das Septin-Familie-Gen oder (-)Protein oder Fragment davon Septin-2, wie in SEQ ID NO: 2 bzw. 1 dargelegt, ist,
insbesondere
A1) wobei das Verfahren weiterhin umfasst:
(i) Messen der Menge von CA125 und/oder HE4 in der Probe;
(ii) Vergleichen der Menge von dem Septin-Familie-Gen oder (-)Protein und der Menge von CA125 und/oder HE4, wie in (i) gemessen, mit einem Referenzwert der Menge von dem Septin-Familie-Gen oder (-)Protein und der Menge von CA125 und/oder HE4, wobei der Referenzwert eine bekannte Klassifizierung einer Beckenmasse, eines Müller'schen Tumors oder eines Müller'schen Krebses darstellt;
(iii) Auffinden einer Abweichung oder keiner Abweichung von dem Septin-Familie-Gen oder (-)Protein und der Menge von CA125 und/oder HE4, wie in (i), von dem Referenzwert in (ii) gemessen; und
(iv) Klassifizieren der Beckenmasse in dem Subjekt, gutartig oder bösartig zu sein, auf Basis des Findens einer Abweichung oder keiner Abweichung; oder
A2) wobei das Verfahren weiter umfasst:
(i) Messen der Menge von (a) einem Septin-Familie-Protein und (b) von HE4 und/oder CA125 in einer Probe von dem Subjekt;
(ii) Einsetzen der in (i) gemessenen Größen in eine Gleichung, in der jeder Menge ein Gewicht gegeben wird; und
(iii) Analysieren, ob der in (ii) erhaltene numerische Wert in einen Bereich von gutartigem bis bösartigem Müller'schen Krebs fällt, wobei die Bereiche von gutartigem, früh bösartigem, grenzwertigem oder bösartigem Müller'schen Krebs etabliert worden sind durch Verwenden derselben Gleichung an Proben von Subjekten wurden, für die zuvor gutartiger bzw. bösartiger Müller'scher Krebs diagnostiziert oder klassifiziert worden ist,
insbesondere wobei der bösartig Müller'sche Krebs früh bösartig oder grenzwertig ist; und/oder
B) wobei das Verfahren weiterhin umfasst: Messen der Menge von ein oder mehreren anderen Biomarkern in der Probe von dem Subjekt, wobei der andere Biomarker ausgewählt ist aus der Gruppe, bestehend aus Mesothelin, CA72-4, Osteopontin, CA125, p21, HE4, Transthyretin, B2-Mikroglobulin, Apolipoprotein A-1, Prä-Albumin, Transferrin, LPA, YKL-40, Follikel-stimulierendem Hormon (FSH), anti-Müller'schem Hormon (AMH) und/oder Inhibin

8. Verfahren nach einem beliebigen der Ansprüche 6-7, weiterhin umfassend morphologisches Analysieren der Beckenmasse durch ein Verfahren, ausgewählt aus der Gruppe, bestehend aus ein oder mehreren von Ultraschall (US), Magnetresonanztomographie (MRT), Computertomographie (CT) und Positronenemissionstomographie-Computertomographie (PET-CT).

9. Verfahren nach einem beliebigen der Ansprüche 1-8,
i) wobei das Verfahren weiterhin Bewerten von Risikofaktoren in dem Subjekt, ausgewählt aus der Gruppe, bestehend aus genetischer Prädisposition aufgrund von Mutationen in der BRCA-Genfamilie, familiärer Prädisposition, Alter, Ernährung, Fettleibigkeit, Fortpflanzungsgeschichte, Menopausenstatus, gynäkologischer Chirurgie, Hormonersatztherapie, Rauchen und Alkoholkonsum umfasst, insbesondere wobei die gynäkologische Chirurgie eine Eileiterunterbindung oder Hysterektomie umfasst; und/oder
ii) wobei das Verfahren zu regelmäßigen Zeitpunkten verwendet wird, um die Expression von a) dem Septin-Familie-Gen oder (-)Protein und/oder b) dem Septin-Familie-Gen oder (-)Protein und dem/den Biomarker(n) in Kombination mit den Risikofaktoren während des Lebens des Subjekts zu verfolgen

10. Verfahren zum Detektieren einer Änderung in der Prognose für ein Subjekt, diagnostiziert mit Müller'schem Krebs, umfassend Messen der Expression von einem Septin-2-Gen oder (-)Protein, wie in SEQ ID NO: 2 bzw. 1 dargelegt, in einer Probe von dem Subjekt während oder nach Behandlung des Müller'schen Krebses, wobei eine Veränderung beim Expressionsspiegel von Septin-2 im Vergleich mit einem Referenzwert für die Expression von einem Septin-2-Gen, (-)Protein oder Fragment davon in einem oder mehreren Subjekten mit gutartigen Tumoren eine Veränderung in der Prognose für das Subjekt anzeigt.

11. Verfahren nach Anspruch 10, wobei das Subjekt prä- oder postmenopausal ist.

12. Verfahren nach einem beliebigen der Ansprüche 1-11,
i) wobei die Probe Blut, Serum, Plasma, Gewebe oder Urin ist; und/oder
ii) wobei der Müller'sche Krebs ausgewählt ist aus der Gruppe, bestehend aus Eierstock-, Eileiter-, primären Peritoneal-, Endometrium- und Uteruskrebsarten, insbesondere wobei der Eierstockkrebs oder Eierstocktumor ausgewählt ist aus der Gruppe, bestehend aus einem oder mehreren epithelialen Karzinomen, bösartigem Geschlechtsstrang-Stromatumor ("malignent sex cord stromal tumor") oder bösartigen Keimzelltumoren, metastasierendem Karzinom, das im Becken oder in den Eierstöcken infiltriert ist, Zystadenom, Fibrom, Thekom, Zystadenofibrom, reifem Teratom, Endometriose, Follikelzyste, Abszess, Struma ovarii, Leydig-Zell-Tumor, parasalpingealer Zyste, Hydrosalpinx, Gelbkörperzyste, klarzelligem Eierstockkarzinom, hämorrhagischer Zyste, Gewebe mit Verkalkungen NOS, nekrotischem Tumor NOS oder Kombinationen davon.

13. Verfahren nach einem beliebigen der Ansprüche 6-12,
i) wobei die Expression von dem Septin-Familie-Protein gemessen wird, insbesondere a) wobei die Expression von dem Septin-Familie-Protein gemessen wird durch Immunohistochemie, ELISA, RIA, Western- oder Immuno-Blotting oder ein anderes auf Antikörper(n) basiertes Verfahren, oder b) wobei die Expression des Septin-Familie-Proteins durch Massenspektrometrie oder (-)Chromatographie gemessen wird; oder
ii) wobei die Expression von dem Septin-2 Familie-Protein durch qRT-PCR, RT-PCR oder ein anderes PCR-basiertes Verfahren, Northern Blotting oder SAGE gemessen wird, oder b) wobei DNA-methylierte Formen von Septin-Familie-Genen, Isoformen von Septin-Familie-Genen, zirkulierende Septin-Familie-DNA, oder microRNA oder Fragmente davon gemessen werden.

14. Mittel zur Verwendung in einem Verfahren zum Behandeln einer proliferativen Krankheit in einem Subjekt, wobei das Verfahren inhibieren der Expression oder Aktivität eines Septin-Familienmitglied-Gens, (-)Proteins oder Fragments davon umfasst,
wobei das Septin-Familie-Gen oder (-)Protein Septin-2, wie in SEQ ID NO: 2 bzw. 1 dargelegt, ist, wobei die proliferative Erkrankung Müller'scher Krebs ist,
i) wobei die Inhibition in einer antitumoralen, antikanzerogenen, antiproliferativen, antiangiogenen oder antilipogenen Wirkung resultiert; und/oder
ii-1) wobei Genexpression des Septin-Familie-Gens durch Verabreichungen einer wirksamen Menge von ein oder mehreren Mitteln inhibiert wird, die spezifisch ein Septin-2-Gen binden, wobei das Mittel aus der Gruppe ausgewählt ist, die aus einem Antisense-Oligonukleotid, einer siRNA und einem Phosphorothio-Oligonukleotid (PTOs) besteht, oder ii-2) wobei Expression von einem Protein der Septin-Familie oder Fragment davon durch Verabreichung einer wirksamen Menge eines Antikörpers inhibiert wird, wobei der Antikörper oder das Fragment spezifisch ein Septin-2-Protein bindet.

15. Kit, umfassend
(i) Mittel, konfiguriert um (a) die Menge an Septin-2-Protein wie in SEQ ID NO: 1 dargelegt, und (b) die Menge von CA125, HE4 und/oder ein oder mehreren anderen Biomarkern in einer Probe von einem Subjekt zu messen, wobei der andere Biomarker ausgewählt ist aus der Gruppe, bestehend aus Mesothelin, CA72-4, Osteopontin, CA125, p21, HE4, Transthyretin, B2-Mikroglobulin, Apolipoprotein A-1, Prä-Albumin, Transferrin, LPA, YKL-40, Follikel-stimulierendem Hormon (FSH), anti-Müller'schem Hormon (AMH) und/oder Inhibin; und
(ii) einen Referenzwert von (a) der Menge an Septin-2 und (b) der Menge an CA125, HE4 und/oder ein oder mehrere von den anderen oben erwähnten Biomarkern, wobei der Referenzwert eine bekannte Klassifikation eines Müller'schen Tumors darstellt.

## Revendications

1. Procédé pour l'évaluation diagnostique d'un cancer mullérien chez un sujet, ledit procédé comprenant l'étape consistant en : la mesure de l'expression d'un gène ou d'une protéine de la famille des septines dans un échantillon provenant du sujet, où le sujet est diagnostiqué avec un cancer mullérien si l'expression du gène ou de la protéine de la famille des septines est supérieure dans l'échantillon à celle dans un ou plusieurs échantillons témoins acquis auprès d'un ou plusieurs sujets sans cancer müllérien,
dans lequel le gène ou la protéine de la famille des septines est celui de la septine-2 tel que présenté dans les SEQ ID NO : 2 et 1 respectivement.

2. Procédé selon la revendication 1,
i) où le procédé comprend en outre l'étape consistant en la mesure de l'expression d'un gène ou d'une protéine d'un ou plusieurs marqueurs biologiques choisis dans le groupe constitué par CA125, p21, HE4, la transthyrétine, LPA, YKL-40 et l'inhibine, où le sujet est diagnostiqué avec un cancer mullérien si a) l'expression du gène ou de la protéine de la famille des septines et b) l'expression dudit gène ou protéine desdits un ou plusieurs marqueurs biologiques sont supérieures dans l'échantillon à celles dans lesdits un ou plusieurs échantillons témoins acquis auprès d'un ou plusieurs sujets sans cancer müllérien, et/ou
ii) où ledit sujet est pré-ménopausé ou ménopausé ; et/ou
iii-1) où ledit sujet a été diagnostiqué comme ayant un cancer mullérien et ledit procédé est utilisé pour déterminer si ledit cancer mullérien a régressé ou progressé ou
iii-2) où ledit sujet n'a pas été antérieurement diagnostiqué comme ayant un cancer mullérien et ledit procédé est utilisé pour évaluer si un cancer mullérien est présent ; et/ou
iv) où l'expression dudit gène ou protéine de la famille des septines dans ledit échantillon est au moins 1 à 500 fois supérieure à celle dans un ou plusieurs échantillons témoins ; et/ou
v) où l'expression dudit gène ou protéine de la famille des septines dans ledit échantillon est au moins 1,3 à 8 fois supérieure à celle dans un ou plusieurs échantillons témoins ; et/ou
vi) où l'expression dudit gène ou protéine de la famille des septines dans ledit échantillon est au moins 6 fois supérieure à celle dans un ou plusieurs échantillons témoins.

3. Procédé selon l'une quelconque des revendications 1 et 2, dans lequel l'expression dudit gène ou protéine de septine-2 dans ledit échantillon est au moins 3 à 6 fois supérieure à celle dans un ou plusieurs échantillons témoins.

4. Procédé selon l'une quelconque des revendications 2 et 3,
i) dans lequel l'expression dudit gène ou protéine de septine-2 dans ledit échantillon est au moins 3 à 6 fois supérieure à celle dans un ou plusieurs échantillons témoins et l'expression dudit gène ou protéine de HE4 dans ledit échantillon est au moins 4 à 8 fois supérieure à celle dans un ou plusieurs échantillons témoins ; et
ii) dans lequel l'expression dudit gène ou protéine de septine-2 dans ledit échantillon est au moins 3 à 6 fois supérieure à celle dans un ou plusieurs échantillons témoins et l'expression d'un ou plusieurs gènes ou protéines de CA125, p21, transthyrétine, LPA, YKL-40 et/ou inhibine dans ledit échantillon est au moins 4 à 8 fois supérieure à celle dans un ou plusieurs échantillons témoins.

5. Procédé selon l'une quelconque des revendications 3 et 4,
i) dans lequel l'expression dudit gène de septine-2 et/ou de ladite protéine de marqueur biologique est mesurée par immunohistochimie, ELISA, RIA, transfert Western ou immunotransfert, ou un autre procédé quantitatif basé sur un anticorps, en particulier où ledit anticorps est un anticorps généré contre le polypeptide codé par la SEQ ID NO : 1, en particulier où l'anticorps est un anticorps monoclonal ou un fragment fonctionnel de celui-ci ; ou
ii) dans lequel l'expression dudit gène de septine-2 et/ou de ladite protéine de marqueur biologique est mesurée par spectrométrie de masse ou chromatographie ; ou
iii) dans lequel l'expression dudit gène de septine-2 et/ou de ladite protéine de marqueur biologique est mesurée par qRT-PCR, RT-PCR ou un autre procédé basé sur une PCR, un transfert Northern, ou SAGE.

6. Procédé pour stratifier un sujet ayant une masse pelvienne pour le risque d'avoir ou de développer un cancer müllérien, le procédé comprenant l'étape consistant en la mesure de l'expression d'un gène ou d'une protéine de la famille des septines dans un échantillon provenant du sujet,
dans lequel le sujet présente un risque accru d'avoir ou de développer un cancer müllérien si l'expression du gène ou de la protéine de la famille des septines est supérieure dans l'échantillon à celle dans un ou plusieurs échantillons témoins acquis auprès d'un ou plusieurs sujets sans cancer müllérien,
dans lequel le gène ou la protéine de la famille des septines est celui de la septine-2 tel que présenté dans les SEQ ID NO : 2 et 1 respectivement,
en particulier
i) où le procédé comprend en outre l'étape consistant en la mesure de l'expression d'un ou plusieurs marqueurs biologiques choisis dans le groupe constitué par CA125, p21, HE4, la transthyrétine, la microglobuline B2, l'apolipoprotéine A-1, la pré-albumine, la transferrine, LPA, YKL-40, la folliculostimuline (FSH), l'hormone antimullérienne (AMH) et l'inhibine, où le sujet présente un risque accru d'avoir ou de développer un cancer mullérien si a) l'expression du gène ou de la protéine de la famille des septines et b) l'expression desdits un ou plusieurs marqueurs biologiques sont supérieures dans l'échantillon à celles dans lesdits un ou plusieurs échantillons témoins acquis auprès d'un ou plusieurs sujets sans cancer mullérien ; et/ou
ii-1) où a) une expression accrue du gène ou de la protéine de la famille des septines ou b) une expression accrue du gène ou de la protéine de la famille des septines et une expression accrue desdits un ou plusieurs marqueurs biologiques signifient que le sujet présente un risque accru d'un ou plusieurs cancers malins ou d'un cancer mullérien malin à un stade précoce, ou ii-2) où a) une expression équivalente du gène de la famille des septines ou b) une expression équivalente du gène de la famille des septines et une expression équivalente desdits un ou plusieurs marqueurs biologiques signifie que la masse pelvienne est bénigne ou est une tumeur à faible potentiel malin (LMP).

7. Procédé pour classer une masse pelvienne chez un sujet risquant d'avoir ou de développer un cancer müllérien, le procédé comprenant l'étape consistant en la mesure de l'expression d'un gène ou d'une protéine de la famille des septines dans un échantillon provenant du sujet, où la masse pelvienne est classée comme étant maligne si l'expression du gène ou de la protéine de la famille des septines est supérieure dans l'échantillon à celle dans un ou plusieurs échantillons témoins acquis auprès de sujets sans cancer müllérien,
dans lequel le gène ou la protéine de la famille des septines est celui de la septine-2 tel que présenté dans les SEQ ID NO : 2 et 1 respectivement,
en particulier
A1) où le procédé comprend en outre les étapes consistant en :
(i) la mesure de la quantité de CA125 et/ou de HE4 dans l'échantillon ;
(ii) la comparaison de la quantité du gène ou de la protéine de la famille des septines et de la quantité de CA125 et/ou de HE4, telle que mesurée en (i), avec une valeur de référence de la quantité du gène ou de la protéine de la famille des septines et de la quantité de CA125 et/ou de HE4, où la valeur de référence représente une classification connue d'une masse pelvienne, d'une tumeur müllérienne, ou d'un cancer müllérien ;
(iii) la découverte d'un écart ou d'une absence d'écart du gène ou de la protéine de la famille des septines et de la quantité de CA125 et/ou de HE4, telle que mesurée en (i), par rapport à ladite valeur de référence en (ii) ; et
(iv) la classification de la masse pelvienne chez ledit sujet comme étant bénigne ou maligne sur la base de ladite découverte d'écart ou d'absence d'écart ; ou
A2) où le procédé comprend en outre les étapes consistant en
(i) la mesure de la quantité de (a) une protéine de la famille des septines et b) HE4 et/ou CA125 dans un échantillon provenant du sujet ;
(ii) l'entrée des quantités mesurées en (i) dans une équation dans laquelle chaque quantité reçoit un poids ; et
(iii) l'analyse quant à savoir si la valeur numérique obtenue en (ii) se trouve dans une plage de cancer müllérien allant de bénin à malin, où lesdites plages de cancer müllérien bénin, malin précoce, limite, ou malin, ont été établies par utilisation de la même équation sur des échantillons provenant de sujets pour lesquels un cancer müllérien respectivement bénin ou malin a été antérieurement diagnostiqué ou classé, et en particulier où ledit cancer müllérien malin est malin précoce, ou limite ; et/ou
B) où le procédé comprend en outre l'étape consistant en : la mesure de la quantité d'un ou plusieurs autres marqueurs biologiques dans l'échantillon provenant dudit sujet, où ledit autre marqueur biologique est choisi dans le groupe constitué par la mésothéline, CA72-4, l'ostéopontine, CA125, p21, HE4, la transthyrétine, la microglobuline B2, l'apolipoprotéine A-1, la préalbumine, la transferrine, LPA, YKL-40, la folliculostimuline (FSH), l'hormone antimüllérienne (AMH) et/ou l'inhibine.

8. Procédé selon l'une quelconque des revendications 6 et 7, comprenant en outre l'étape consistant en l'analyse morphologique de la masse pelvienne par un ou plusieurs procédés choisis dans le groupe constitué par les ultrasons (US), l'imagerie par résonance magnétique (IRM), la tomodensitométrie (CT), et la tépographie-tomodensitométrie (PET-CT).

9. Procédé selon l'une quelconque des revendications 1 à 8,
i) où le procédé comprend en outre la détermination de facteurs de risque chez le sujet, choisis dans le groupe constitué par une prédisposition génétique due à des mutations dans la famille des gènes BRCA, une prédisposition familiale, l'âge, le régime alimentaire, l'obésité, l'historique de reproduction, le statut de la ménopause, une opération chirurgicale gynécologique, un traitement substitutif hormonal, le tabagisme et l'alcoolisme, en particulier où l'opération chirurgicale gynécologique comprend une ligature des trompes ou une hystérectomie ; et/ou
ii) où le procédé est utilisé à des points de temps réguliers pour suivre l'expression de a) le gène ou la protéine de la famille des septines et/ou b) le gène ou la protéine de la famille des septines et ledit ou lesdits marqueurs biologiques en combinaison avec les facteurs de risques au cours de la vie du sujet.

10. Procédé pour détecter un changement dans le pronostic pour un sujet diagnostiqué avec un cancer müllérien, comprenant l'étape consistant en la mesure de l'expression d'un gène ou d'une protéine de septine-2 comme indiqué dans les SEQ ID NO : 2 et 1 respectivement, dans un échantillon provenant du sujet pendant ou après traitement du cancer müllérien, où un changement du niveau d'expression de septine-2 en comparaison avec une valeur de référence pour l'expression d'un gène ou d'une protéine de septine-2 ou d'un fragment de celui-ci chez un ou plusieurs sujets ayant des tumeurs bénignes indique un changement du pronostic pour le sujet.

11. Procédé selon la revendication 10, dans lequel le sujet est pré-ménopausé ou ménopausé.

12. Procédé selon l'une quelconque des revendications 1 à 11,
i) dans lequel ledit échantillon est du sang, du sérum, du plasma, du tissu, ou de l'urine ; et/ou
ii) dans lequel ledit cancer müllérien est choisi dans le groupe constitué par les cancers des ovaires, des trompes de Fallope, du péritoine primaire, de l'endomètre et de l'utérus, en particulier où ledit cancer ovarien ou ladite tumeur ovarienne est un ou plusieurs choisis dans le groupe constitué par un carcinome épithélial, une tumeur maligne du stroma des cordons sexuels, une tumeur maligne à cellules germinales, un carcinome métastasique infiltré dans le pelvis ou dans les ovaires, un cystadénome, un fibrome, un thécome, un cystadénofibrome, un tératome mature, une endométriose, un kyste folliculaire, un abcès, un struma ovarii, une tumeur à cellules de Leydig, un kyste parasalpingé, un hydrosalpinx, un kyste du corps jaune, un carcinome ovarien à cellules claires, un kyste hémorragique, un tissu avec des calcifications NOS, une tumeur nécrotique NOS ou leurs combinaisons.

13. Procédé selon l'une quelconque des revendications 6 à 12,
i) dans lequel l'expression d'une protéine de la famille des septines est mesurée, en particulier a) où l'expression de la protéine de la famille des septines est mesurée par immunohistochimie, ELISA, RIA, transfert Western ou immunotransfert, ou un autre procédé quantitatif basé sur un anticorps, ou b) où l'expression de la protéine de la famille des septines est mesurée par spectrométrie de masse ou chromatographie ; ou
ii) dans lequel l'expression d'une protéine de la famille des septines est mesurée, en particulier a) où l'expression de la protéine de la famille des septines est mesurée par qRT-PCR, RT-PCR ou un autre procédé basé sur une PCR, un transfert Northern, ou SAGE, ou b) où des formes méthylées d'ADN de gènes de la famille des septines, des isoformes de gènes de la famille des septines, de l'ADN de la famille des septines en circulation, ou du microARN ou des fragments de ceux-ci, sont mesurés.

14. Agent pour une utilisation dans une méthode de traitement d'une maladie proliférative chez un sujet, la méthode comprenant l'inhibition de l'expression ou de l'activité d'un gène ou d'une protéine membre de la famille des septines, ou d'un fragment de celui-ci ;
où le gène ou la protéine de la famille des septines est celui de la septine-2 tel que présenté dans les SEQ ID NO : 2 et 1 respectivement, où la maladie proliférative est un cancer müllérien,
i) où ladite inhibition a pour résultat un effet anti-tumoral, anticancéreux, antiprolifératif, anti-angiogénique, ou anti-lipogénique ; et/ou
ii-1) où l'expression du gène membre de la famille des septines est inhibée par l'administration d'une quantité efficace d'un ou plusieurs agents qui se lient spécifiquement au gène de septine-2, où ledit agent est choisi dans le groupe constitué par un oligonucléotide antisens, un ARNsi, et un phosphorothio-oligonucléotide (PTO), ou ii-2) où l'expression de la protéine membre de la famille des septines ou de son fragment est inhibée par l'administration d'une quantité efficace d'un anticorps ou d'un fragment de celui-ci, où ledit anticorps ou fragment de celui-ci se lie spécifiquement à la protéine septine-2.

15. Kit comprenant
(i) un moyen configuré pour mesurer (a) la quantité de la protéine de septine-2 telle que présentée dans la SEQ ID NO : 1 et (b) la quantité de CA125, de HE-4 et/ou d'un ou plusieurs autres marqueurs biologiques dans un échantillon provenant d'un sujet, où ledit autre marqueur biologique est choisi dans le groupe constitué par la mésothéline, CA72-4, l'ostéopontine, CA125, p21, HE4, la transthyrétine, la microglobuline B2, l'apolipoprotéine A-1, la pré-albumine, la transferrine, LPA, YKL-40, la folliculostimuline (FSH), l'hormone antimüllérienne (AMH) et/ou l'inhibine ; et
(ii) une valeur de référence de (a) la quantité de septine-2 et (b) la quantité de CA125, de HE4 et/ou d'un ou plusieurs des autres marqueurs biologiques mentionnés ci-dessus, où ladite valeur de référence représente une classification connue d'une tumeur müllérienne.
